(19) Europäisches Patentamt
European Patent Office
Office européen des brevets

(11) EP 2 065 379 A1

(12) EUROPEAN PATENT APPLICATION
published in accordance with Art. 153(4) EPC

(43) Date of publication:
**03.06.2009 Bulletin 2009/23**

(21) Application number: **07829601.9**

(22) Date of filing: **11.10.2007**

(51) Int Cl.:
***C07D 401/12*** (2006.01) ***A61K 31/4439*** (2006.01)
***A61K 31/549*** (2006.01) ***A61P 1/04*** (2006.01)
***A61P 31/04*** (2006.01) ***A61P 43/00*** (2006.01)
***C07D 405/14*** (2006.01) ***C07D 493/10*** (2006.01)
***C07D 513/14*** (2006.01) ***C07D 519/00*** (2006.01)

(86) International application number:
**PCT/JP2007/069863**

(87) International publication number:
**WO 2008/047681 (24.04.2008 Gazette 2008/17)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **13.10.2006 JP 2006280285**

(71) Applicant: **Eisai R&D Management Co., Ltd.**
**Tokyo 112-8088 (JP)**

(72) Inventors:
- **UEDA, Masato**
**Tsukuba-shi**
**Ibaraki 300-2635 (JP)**
- **IIDA, Daisuke**
**Tsukuba-shi**
**Ibaraki 300-2635 (JP)**
- **KIKUCHI, Sachiko**
**Tsukuba-shi**
**Ibaraki 300-2635 (JP)**
- **GOTODA, Masaharu**
**Tsukuba-shi**
**Ibaraki 300-2635 (JP)**

(74) Representative: **HOFFMANN EITLE**
**Patent- und Rechtsanwälte**
**Arabellastrasse 4**
**81925 München (DE)**

# (54) BENZIMIDAZOLE COMPOUND HAVING GASTRIC ACID SECRETION INHIBITORY ACTIVITY

(57) The present invention provides a compound that has superior gastric acid secretion inhibitory action, ample long-lasting gastric acid secretion inhibitory action, and is able to maintain intragastric pH at a high level for an extended period of time, making it useful as a therapeutic or prophylactic agent for diseases or symptoms caused by gastric acid, as well as an active form of this compound (form to which the compound is converted in the body following administration thereof) in the form of a compound represented by general formula (1 a) (wherein R2 represents a group represented by formula (I) or the like, and R1, R3, W1 and $X^-$ are as defined in the description).

FIG. 1

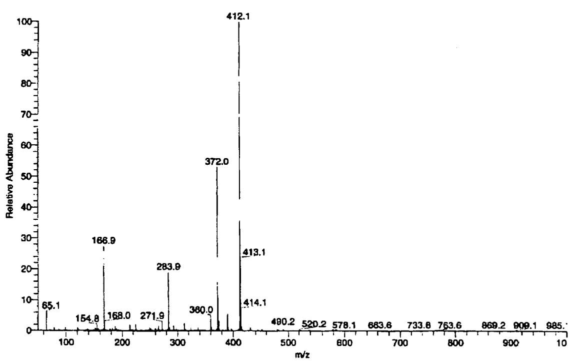




EP 2 065 379 A1

## Description

Technical Field

The present invention relates to benzimidazole compounds or salts thereof which are useful as an inhibitor of secretion of gastric acid.

Background Art

Peptic ulcers such as gastric ulcers and duodenal ulcers are believed to occur as a result of a disruption of the balance between offensive factors such as acid and pepsin and defensive factors such as mucous and blood flow, thereby causing auto-digestion.

The treatment of peptic ulcers involves internal medication as a general rule, and various drug therapies have been attempted. Recently in particular, drugs that inhibit auto-digestion as a result of suppressing secretion of gastric acid by specifically inhibiting H+,K+-ATPase, which is an enzyme present in the parietal cells that governs the final step of gastric acid secretion, have been developed and used clinically, examples of which may include omeprazole, esomeprazole, pantoprazole, lansoprazole, rabeprazole or the like.

Although these drugs have superior therapeutic effects, there is a further need for a drug having longer-lasting gastric acid secretion inhibitory action that is safer and has suitable physicochemical stability. In particular, the cure rate of reflux esophagitis has been suggested to improve by maintaining a higher pH in the stomach for an extended period of time (see, for example, Non-Patent Document 1).

Although the compounds relating to the present invention in particular are described in Patent Document 1 or Patent Document 2, the chemical structures of the specific compounds detailed in these publications and the specific compound of the present application are clearly different.

The gastric acid secretion inhibitory action of these benzimidazole derivatives having gastric acid secretion inhibitory action has previously been found to not only be due to these compounds themselves, but also due to conversion products thereof (see, for example, Patent Document 3). Namely, these benzimidazole derivatives are weakly basic compounds that gather at acidic sites after being absorbed into the body. The parietal cells containing (H+ + K+)-ATPase are said to be the only cells in the body that exhibit a low pH value. Benzimidazole derivatives are chemically converted to sulfenamide derivatives by acid produced by enzyme in close proximity thereto, and these sulfenamide forms are considered to demonstrate (H+ + K+)-ATPase inhibitory action (see, for example, Non-Patent Document 2).

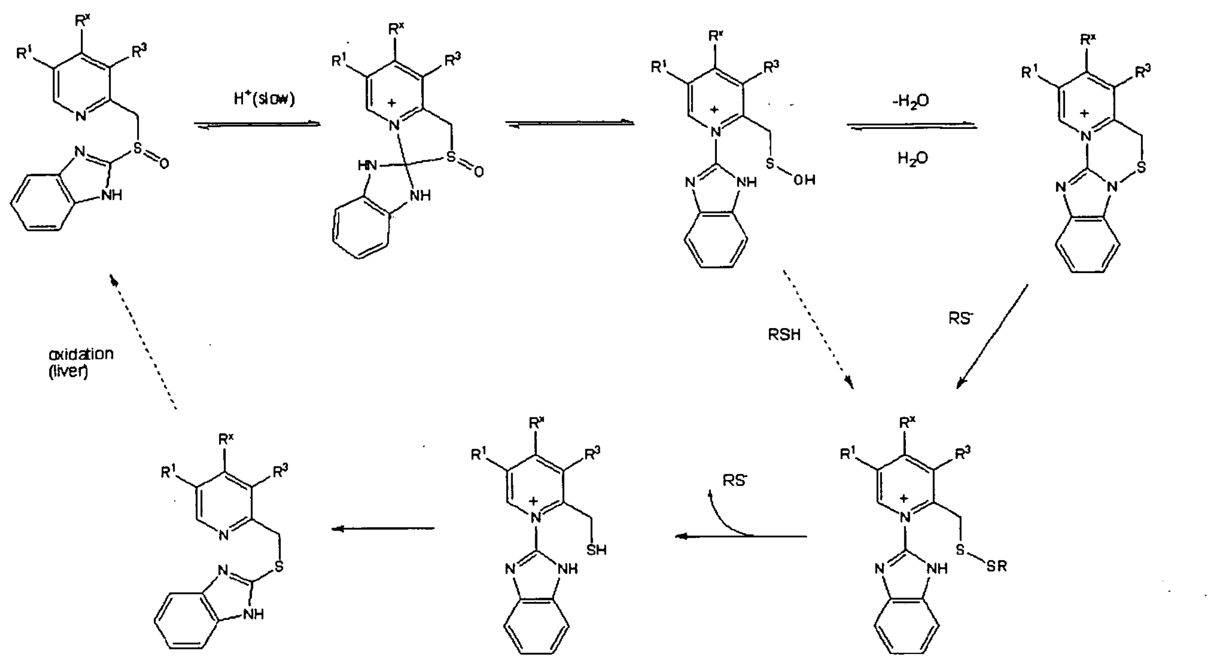

Patent Document 1: International Publication WO 91/19712 pamphlet
Patent Document 2: Japanese Patent Application Laid-open No. S59-181277
Patent Document 3: Japanese Patent Application Laid-open No. S61-7281
Non-Patent Document 1: Digestion 1992; 51 (suppl. 1): 59-67
Non-Patent Document 2: Journal of Medicinal Chemistry 1986, 29, 1327-1329

Disclosure of Invention

Problems to be Solved by the Invention

An object of the present invention is to provide novel compounds having superior gastric acid secretion inhibitory action and ample long-lasting gastric acid secretion inhibitory action and able to maintain a high intragastric pH for an extended period of time that is useful as a therapeutic or prophylactic agent of diseases or symptoms caused by gastric acid, and to provide active forms of the novel compounds (converted forms that are converted in the body following administration) having ample long-lasting gastric acid secretion inhibitory action that are able to maintain a high intragastric pH for an extended period of time.

Means for Solving the Problems

As a result of conductive extensive studies to solve the above problems, the inventors of the present invention found that benzimidazole compounds having a novel chemical structure have superior gastric acid secretion inhibitory action, ample long-lasting gastric acid secretion inhibitory action, are able to maintain a high intragastric pH for an extended period of time, and are useful as a therapeutic or prophylactic agent for reflux esophagitis, symptomatic reflux esophagitis, gastric ulcer or duodenal ulcer in particular, thereby leading to completion of the present invention.
Namely, the present invention provides <1> a compound represented by the following general formula (1 a) and <2> a compound represented by the following general formula (1b), or a salt thereof.

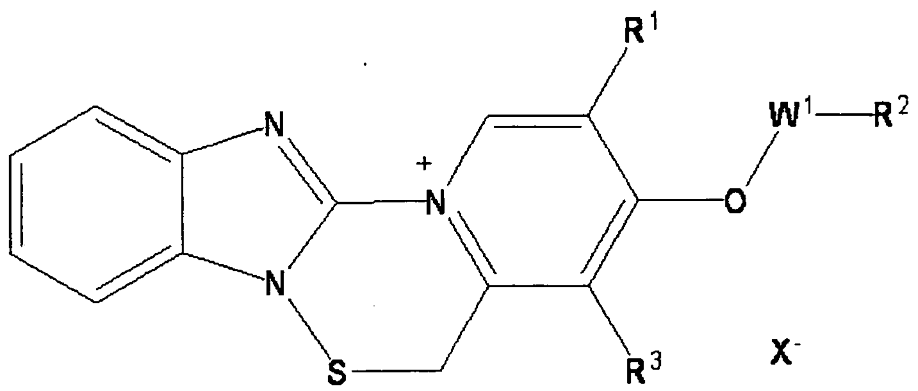

(1 a)

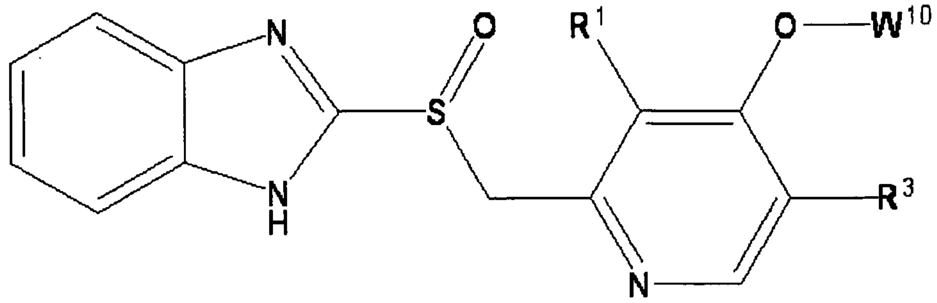

(1b)

In general formula (1a) above, R1 and R3 may be the same or different and represent a hydrogen atom or a C1-C6 alkyl group, R2 represents a group represented by the following formula:

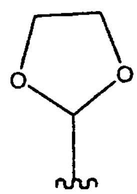
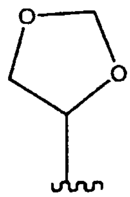
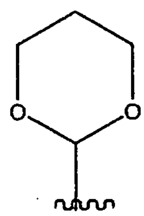
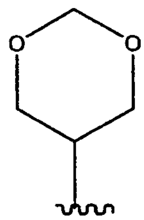
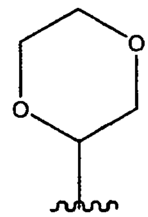
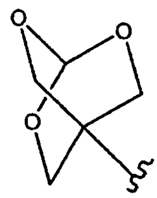

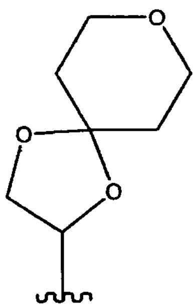
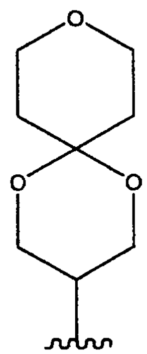
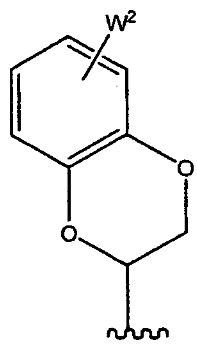
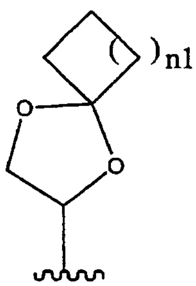
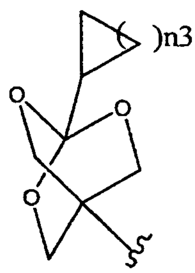

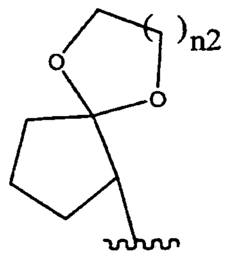
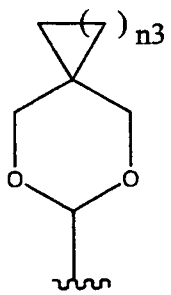
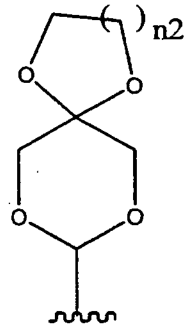
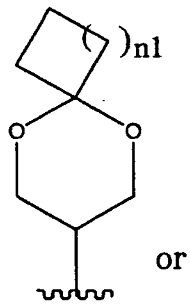
or
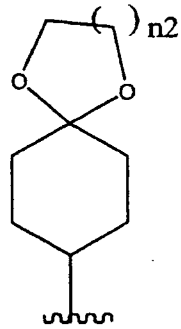

in which the group may have one to four groups selected from group A1 below, or a C2-C6 alkyl group having one to three hydroxyl groups, W1 represents a single bond or a linear or branched C1-C8 alkylene group, W2 represents a hydrogen atom, C1-C6 alkyl group or halogen atom (provided that one to three same or different W2 groups can be present on a benzene ring), n1 represents 1 to 5, n2 represents 1 to 4, n3 represents 1 to 6, and X- represents $Cl^-$, $Br^-$, $I^-$, $BF_4^-$, $PF_6^-$, $HSO_4^-$, $SO_4^{2-}$, $CH_3SO_3^-$, 4-Me-Ph-$SO_3$-, $PO_4^{3-}$, $ClO_4^-$ or $AuCl_4^-$.

<Group A1>

a halogen atom, a C1-C6 alkyl group, a C1-C6 alkoxy group, a C1-C6 haloalkyl group, a C1-C6 alkoxy-C1-C6 alkyl group and a hydroxyl group.

In addition, in general formula (1 b) above, R1 and R3 may be the same or different and represent a hydrogen atom or C1-C6 alkyl group, and W10 represents a group represented by the following formula:

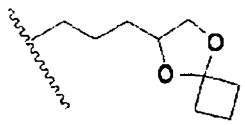
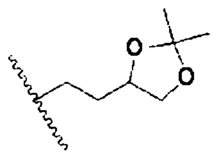
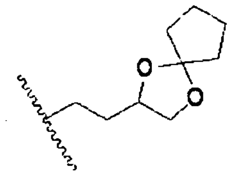
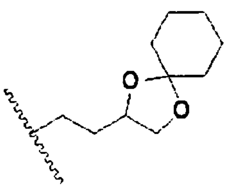
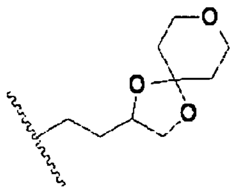
or
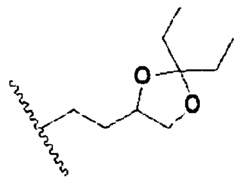

or a C2-C6 alkyl group having one to three hydroxyl groups.

In addition, the present invention provides a medicament containing the compound represented by general formula (1a) above, general formula (1b) above or the salt thereof.

Moreover, the present invention provides a gastric acid secretion inhibitor containing the compound represented by general formula (1a) above, general formula (1 b) above or the salt thereof.

In addition, the present invention provides a pharmaceutical composition containing the compound represented by general formula (1a) above, general formula (1b) above or the salt thereof, or a use of the compound represented by general formula (1 a) above, general formula (1b) above or the salt thereof for producing a pharmaceutical composition.

Moreover, the present invention provides a therapeutic or prophylactic agent containing the compound represented by general formula (1 a) above, general formula (1 b) above or the salt thereof for diseases or symptoms caused by gastric acid, and more specifically, gastric ulcer, duodenal ulcer, anastomotic ulcer, reflex esophagitis (including that having repeated recurrence or relapse), Zollinger-Ellison syndrome, symptomatic reflux esophagitis, endoscopy-negative reflux esophagitis, non-erosive reflux esophagitis, gastroesophageal reflux disease, non-ulcer dyspepsia (NUD), laryngopharyngeal abnormalities, Barrett's esophagus, NSAID ulcer, gastritis, gastric hemorrhage, hemorrhagic gastritis, gastrointestinal hemorrhage, peptic ulcer, hemorrhagic ulcer, stress ulcer, gastric hyperacidity, dyspepsia, gastroparesis, geriatric ulcer, refractory ulcer, acute gastric mucosal lesion, heartburn, heartburn during sleep apnea, grinding of teeth, gastralgia, indigestion, upset stomach, nausea, temporomandibular joint disorder or gastric erosion.

In a compound represented by general formula (1a) above, R2 represents preferably a group represented by the following formula:

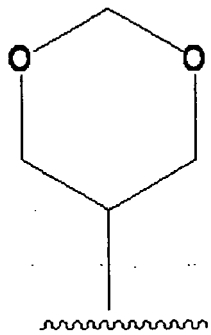

in which the group may have one or two C1-C6 alkyl groups, or a C3-C6 alkyl group having two hydroxyl groups.

In the compound represented by general formula (1a) above, formula W1-R2 preferably represents the formula below:

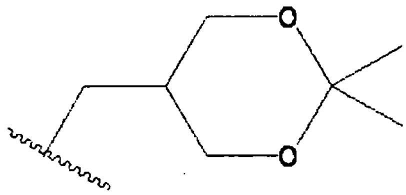

or

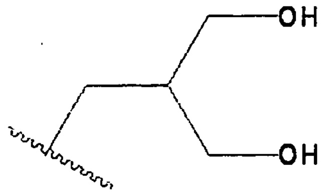

In the compound represented by general formula (1a) above, R1 and R3 preferably represents methyl groups.

In the compound represented by general formula (1a) above, $X^-$ preferably represents $Cl^-$, $Br^-$, $BF_4^-$, $PF_5^-$, $PO_4^{3-}$ or $ClO_4^-$ .

Examples of the compound according to the present invention or the salt thereof may include
2-[[[4-[3-(5,8-dioxaspiro[3,4]octa-6-yl)propoxy]-3-methylpyridin-2-yl]methyl]sulfinyl]-1 *H*-benzimidazole,
2-[[[4-[2-(5,8-dioxaspiro[3,4]octa-6-yl)ethoxy]-3-methylpyridin-2-yl]methyl] sulfinyl]-1*H*-benzimidazole,
2-[[[4-[2-(1,4-dioxaspiro[4,4]nona-2-yl)ethoxy]-3-methylpyridin-2-yl]methyl]sulfinyl]-1*H* -benzimidazole,
2-[[[4-[2-(1,4-dioxaspiro[4,5]deca-2-yl)ethoxy]-3-methylpyridin-2-yl]methyl] sulfinyl]-1*H*-benzimidazole,
2-[[[3-methyl-4-[2-(1,4,8-trioxaspiro[4,5] deca-2-yl)ethoxy]-pyridin-2-yl]methyl]sulfinyl]-1*H*-benzimidazole,
2-[[[4-[2-(2,2-diethyl-1,3-dioxolan-4-yl)ethoxy]-3-methylpyridin-2-yl]methyl] sulfinyl]-1*H*-benzimidazole, and 2-[[[4-[3-hydroxy-2-(hydroxymethyl)propoxy]-3,5-dimethylpyridin-2-yl]methyl]sulfinyl]-1*H*-benzimidazole.

Preferable examples of "diseases or symptoms caused by gastric acid" may include gastric ulcer, duodenal ulcer, anastomotic ulcer, reflex esophagitis, Zollinger-Ellison syndrome, symptomatic reflux esophagitis, endoscopy-negative reflux esophagitis, non-erosive reflux esophagitis and acute gastric mucosal lesion, more preferable examples include reflux esophagitis, symptomatic reflux esophagitis, gastric ulcer and duodenal ulcer, and even more preferable examples may include (1) reflux esophagitis or symptomatic reflux esophagitis, and (2) gastric ulcer or duodenal ulcer.

On the other hand, the present invention provides a disinfectant or disinfection assistant for Helicobacter pylori containing the compound represented by general formula (1 a) above, the compound represented by general formula (1 b) above or the salt thereof.
Furthermore, the above-mentioned "prophylactic agent" may include maintenance therapeutic agents and relapse prophylactics administered following treatment in addition to those administered prior to the onset of a disease or symptoms.
In addition, the above-mentioned "disinfection assistant" refers to a drug that provides an environment that enables a disinfectant, which has difficulty in demonstrating effects under acidic conditions, to demonstrate effects.

The following provides a detailed explanation of the present invention by explaining the meanings of terms, symbols and the like used in the present description.
The term "C1-C6 alkyl group" used in the present Specification refers to a linear or branched alkyl group having 1 to 6 carbons such as a methyl group , an ethyl group, a n-propyl group, an isopropyl group, a n-butyl group, an isobutyl group, a s-butyl group, a t-butyl group, a n-pentyl group, an isopentyl group, a 2-methylbutyl group, a neopentyl group, a 1-ethylpropyl group, a n-hexyl group, an isohexyl group, a 3-methylpentyl group, a 2-methylpentyl group, a 1-methylpentyl group, a 3,3-dimethylbutyl group, a 2,2-dimethylbutyl group, a 1,1-dimethylbutyl group, a 1,2-dimethylbutyl group, a 1,3-dimethylbutyl group, a 2,3-dimethylbutyl group, a 1-ethylbutyl group, a 2-ethylbutyl group or the like.

The term "C2-C6 alkyl group" used in the present Specification refers to a linear or branched alkyl group having 2 to 6 carbons such as an ethyl group, a n-propyl group, an isopropyl group, a n-butyl group, an isobutyl group, a s-butyl group, a t-butyl group, a n-pentyl group, an isopentyl group, a 2-methylbutyl group, a neopentyl group, a 1-ethylpropyl group, a n-hexyl group, an isohexyl group, a 3-methylpentyl group, a 2-methylpentyl group, a 1-methylpentyl group, a 3,3-dimethylbutyl group, a 2,2-dimethylbutyl group, a 1,1-dimethylbutyl group, a 1,2-dimethylbutyl group, a 1,3-dimethylbutyl group, a 2,3-dimethylbutyl group, a 1-ethylbutyl group, a 2-ethylbutyl group or the like.

The term "C3-C6 alkyl group" used in the present Specification refers to a linear or branched alkyl group having 3 to 6 carbons such as an n-propyl group, an isopropyl group, a n-butyl group, an isobutyl group, a s-butyl group, a t-butyl group, a n-pentyl group, an isopentyl group, a 2-methylbutyl group, a neopentyl group, a 1-ethylpropyl group, a n-hexyl group, an isohexyl group, a 3-methylpentyl group, a 2-methylpentyl group, a 1-methylpentyl group, a 3,3-dimethylbutyl group, a 2,2-dimethylbutyl group, a 1,1-dimethylbutyl group, a 1,2-dimethylbutyl group, a 1,3-dimethylbutyl group, a 2,3-dimethylbutyl group, a 1-ethylbutyl group, a 2-ethylbutyl group or the like.

The term "halogen atom" used in the present Specification refers to a fluorine atom, a chlorine atom, a bromine atom or an iodine atom.

The term "C1-C6 alkoxy group" used in the present Specification refers to a linear or branched alkoxy group having 1 to 6 carbons such as an ethoxy group, a methoxy group, a propoxy group, an isopropoxy group, a butoxy group, an isobutoxy group, a s-butoxy group, a t-butoxy group, a pentoxy group, an isopentoxy group, a 2-methylbutoxy group, a neopentoxy group, a hexyloxy group, a 4-methylpentoxy group, a 3-methylpentoxy group, a 2-methylpentoxy group, a 3,3-dimethylbutoxy group, a 2,2-dimethylbutoxy group, a 1,1-dimethylbutoxy group, a 1,2-dimethylbutoxy group, a 1,3-dimethylbutoxy group, a 2,3-dimethylbutoxy group or the like.

The term "C1-C6 haloalkyl group" used in the present Specification refers to an above-mentioned C1-C6 alkyl group substituted with one to five of the above-mentioned halogen atoms, examples of which may include a monofluoromethyl group, a monochloromethyl group, a monobromomethyl group, a monoiodomethyl group, a difluoromethyl group, a dichloromethyl group, a dibromomethyl group, a diiodomethyl group, a trifluoromethyl group, a trichloromethyl group, a tribromomethyl group, a triiodomethyl group, a 1-fluoroethyl group, a 2-fluoroethyl group, a 2,2,2-trifluoroethyl group, a 1-chloroethyl group, a 2-chloroethyl group, a 2,2,2-trichloroethyl group, a 1-fluoropropyl group, a 2-bromopropyl group, a 1-bromobutyl group, a 1-chloropentylgroup, a 1-fluorohexyl group or the like.

The term "C1-C6 haloalkoxy group" used in the present Specification refers to an above-mentioned C1-C6 alkoxy group substituted with one to five of the above-mentioned halogen atoms, examples of which may include a monofluoromethoxy group, a monochloromethoxy group, a monobromomethoxy group, a monoiodomethoxy group, a difluoromethoxy group, a dichloromethoxy group, a dibromomethoxy group, a diiodomethoxy group, a trifluoromethoxy group, a trichloromethoxy group, a tribromomethoxy group, a triiodomethoxy group, a 1-fluoroethyloxy group, a 2-fluoroethyloxy group, a 2,2,2-trifluoroethyloxy group, a 1-chloroethyloxy group, a 2-chloroethyloxy group, a 2,2,2-trichloroethyloxy group, a 1-fluoropropyloxy group, a 2-bromopropyloxy group, a 1-bromobutyloxy group, a 1-chloropentyloxy group, a 1-fluorohexyloxy group or the like.

The term "C1-C6 alkoxy-C1-C6 alkyl group" used in the present Specification refers to an above-mentioned C1-C6 alkyl group mono-substituted with an above-mentioned C1-C6 alkoxy group, examples of which may include a methoxymethyl group, an ethoxymethyl group, a propoxymethyl group, a 2-methoxyethyl group, a 2-ethoxyethyl group, a 1-methoxyethyl group, a 3-methoxypropyl group, a 3-ethoxypropyl group, a 4-methoxybutyl group, a 4-ethoxybutyl group, a 4-propoxybutyl group, a 5-methoxypentyl group, a 5-ethoxypentyl group, a 5-propoxypentyl group, a 6-methoxyhexyl group, a 6-ethoxyhexyl group or the like.

The term "linear or branched C1-C8 alkylene group" used in the present Specification refers to a group such as a methylene group, an ethylene group, a trimethylene group, a tetramethylene group, a pentamethylene group, a hexamethylene group, a propylene (1-methylethylene) group, a 1-methyltrimethylene group, a 2-methyltrimethylene group or the like.

In the present description, although the structural formula of the compounds may represent a fixed isomer for the sake of convenience, the present invention may include all isomers such as geometric isomers or optical isomers as well as isomer mixtures formed in terms of the structure of the compounds, are not limited by the description of the formula for the sake of convenience, and may be any one isomer or a mixture thereof. Thus, although optically active forms and racemic forms may exist for the compounds according to the present invention, there are no limitations thereon in the present invention, and all such optically active forms and racemic forms are included. In addition, although crystal polymorphism may also exist, there are similarly no limitations thereon, such crystal polymorphisms may be single crystals or mixtures, and both anhydrides and solvates (and particularly hydrates) are included in the present invention.

Moreover, compounds that form the compound (1 a) or the compound (1b) of the present invention by undergoing metabolism such as oxidation, reduction, hydrolysis or conjugation in the body (so-called prodrugs) are also included in the present invention.

The compounds according to the present invention forms a salt at an NH group at position 1 or position 3 in the benzimidazole backbone in the above-mentioned general formula (1b). There are no particular limitations on that

"salt" provided it is pharmaceutically acceptable, examples of which may include salts of inorganic bases and organic bases.

Preferable examples of salts of inorganic bases may include alkaline metal salts such as sodium salts, potassium salts or lithium salts; alkaline earth metal salts such as calcium salts or magnesium salts; transition metal salts such as zinc salts, aluminum salts and ammonium salts, while preferable examples of salts of organic bases may include diethylamine salts, diethanolamine salts, meglumin salts and N,N'-dibenzylethylenediamine salts.

In the present description, there are no particular limitations on a "solvate" provided it is pharmaceutically acceptable, specific examples of which may include hydrates, ethanolates and acetonates, with hydrates being used preferably.

Advantageous Effects of the Invention

Since the compound according to the present invention have superior gastric acid secretion inhibitory action, ample long-lasting gastric acid secretion inhibitory action, is able to maintain a high intragastric pH for an extended period of time, is safe and has suitable physicochemical stability, it is useful as a pharmaceutical, and particularly as a therapeutic or prophylactic agent of diseases or symptoms caused by gastric acid, as well as a disinfectant or disinfection assistant for Helicobacter pylori.

Brief Description of Drawings

FIG. 1 shows a graph showing the mass spectrum results measured immediately after (within 1 minute) of the addition of hydrochloric acid to a sodium salt of an optical isomer having a short retention time of 2-[[[4-(2,2-dimethyl-1,3-dioxan-5-yl)methoxy]-3,5-dimethylpyridin-2-yl]methyl]sulfinyl]-1*H*-benzimidazole.

Best Mode for Carrying Out the Invention

The following embodiments are examples illustrative for explaining the present invention and are not intended to limit the present invention to these embodiments. The present invention can be carried out in various forms without departing from the gist thereof.

Compounds according to the present invention can be produced in accordance with the methods described below. However, methods for producing the compounds according to the present invention are not limited thereto. A compound (1) according to the present invention can be produced according to the following Method A.

Method A

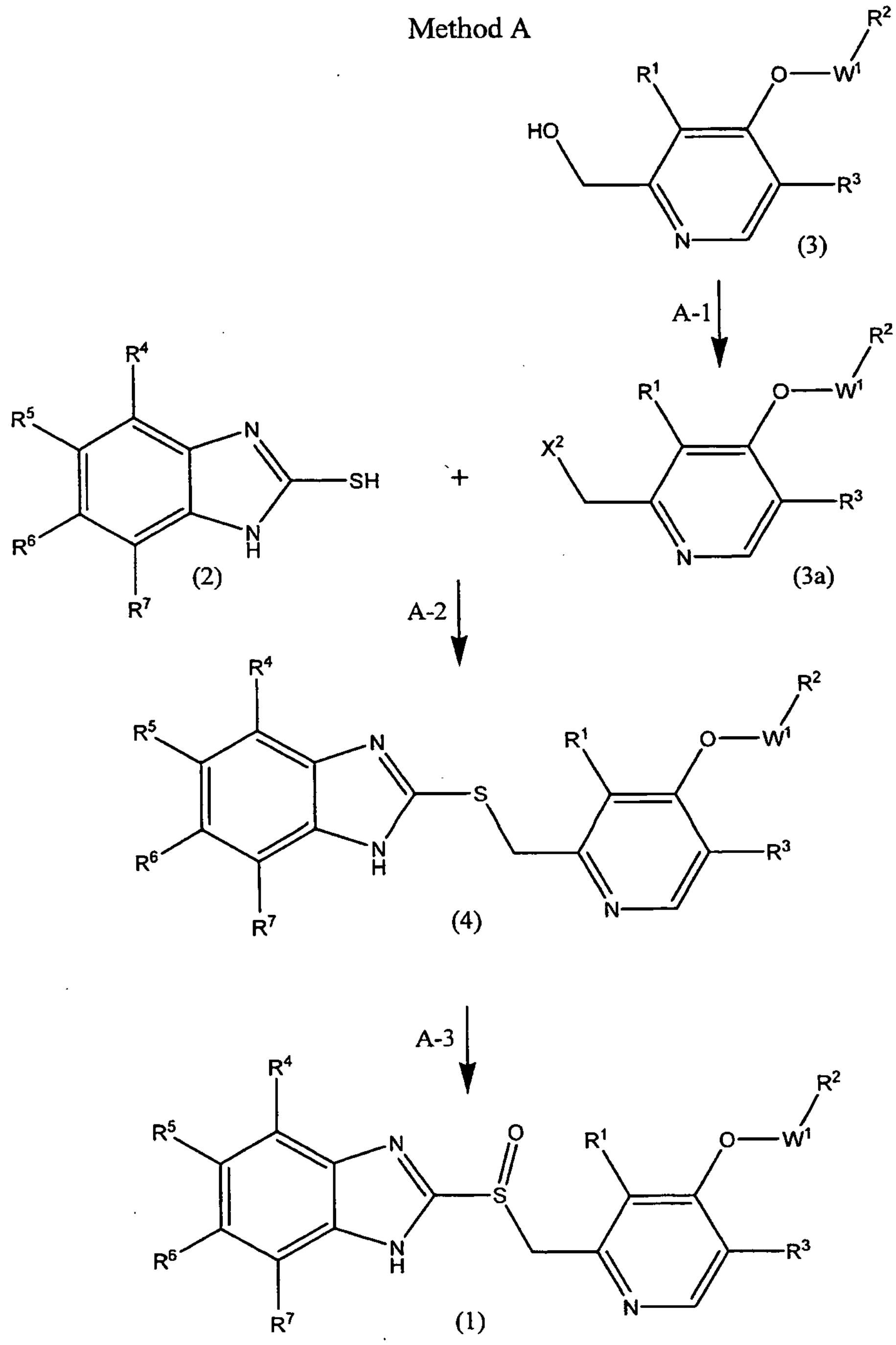

(3) (2) (3a) (4) (1)

In the above formulas, R1, R2, R3 and W1 are the same as defined above, while X2 represents a leaving group. In the above formulas, R4, R5, R6 and R7 may be the same or different and represent a hydrogen atom, a hydroxyl group, a halogen atom, a C1-C6 alkyl group, a C1-C6 haloalkyl group, a C1-C6 alkoxy group or C1-C6 haloalkoxy group, or R5 and R6 together represent a methylenedioxy group or an ethylenedioxy group.

Examples of the leaving groups of X2 may include sulfonyloxy groups such as methanesulfonyloxy, p-toluenesulfonyloxy or trifluoromethanesulfonyloxy, halogen groups such as chlorine, bromine or iodine; and acyloxy groups such as acetyloxy, trifluoroacetyloxy or propionyloxy, with methanesulfonyloxy, p-toluenesulfonyloxy, chlorine or acetyloxy group being used preferably.

The following provides an explanation of each step of Method A.

(Step A-1) Leaving Group Introduction or Halogenation

(1) Leaving Group Introduction Reaction

This step is a step for producing compound (3a) or a salt thereof by reacting compound (3) with a leaving group introducing agent in the absence of a solvent or in an inert solvent and in the presence of a base.
There are no particular limitations on the solvent used provided it dissolves the starting raw materials to a certain extent and does not impair the reaction, examples of which may include halogenated hydrocarbons such as chloroform, dichloromethane, 1,2-dichloroethane or carbon tetrachloride; aromatic hydrocarbons such as benzene, toluene or benzotrifluoride; ethers such as diethyl ether, diisopropyl ether, tetrahydrofuran, dioxane, dimethoxyethane or diethylene glycol dimethyl ether; amides such as formamide, N,N-dimethylformamide, N,N-dimethylacetoamide or hexamethyl phosphoric triamide; pyridine and mixed solvents thereof, with halogenated hydrocarbons, ethers or mixed solvents of ethers and aromatic hydrocarbons being used preferably, and dichloromethane, tetrahydrofuran or a mixed solvent of tetrahydrofuran and toluene being used most preferably.
Examples of the leaving group introducing agents used may include sulfonylating agents such as methanesulfonyl chloride, p-toluenesulfonyl chloride, trifluoromethanesulfonyl chloride or N-phenyl-bis(trifluoromethanesulfonimide), with methanesulfonyl chloride or p-toluenesulfonyl chloride being used preferably, and methanesulfonyl chloride being used most preferably.
Examples of bases used may include tertiary alkylamines such as trimethylamine or triethylamine; pyridine, calcium carbonate, sodium carbonate, sodium hydroxide and potassium hydroxide, with triethylamine or sodium hydroxide being used preferably, and triethylamine being used most preferably.
Although varying according to the starting raw materials, the solvent, the leaving group introducing agent and the base, the reaction temperature is generally from -50 to 100°C and preferably from -20 to 40°C. Although varying according to the starting raw materials, the solvent, the leaving group introducing agent, the base and the reaction temperature, the reaction time is generally from 15 minutes to 12 hours and preferably from 30 minutes to 2 hours.
Furthermore, the compounds of the present step can be used as is in the next step without particularly having to be isolated.

(2) Halogenation Reaction (Using the Typical Example of Chlorination Reaction)

This step is a step for producing compound (3a) by reacting a chlorinating agent with compound (3) in the absence of solvent or in an inert solvent and in the presence or absence of base.
There are no particular limitations on the solvent used provided it dissolves the starting raw materials to a certain extent and does not impair the reaction, examples of which may include halogenated hydrocarbons such as chloroform, dichloromethane, 1,2-dichloroethane or carbon tetrachloride; aromatic hydrocarbons such as benzene, toluene or benzotrifluoride; and ethers such as diethyl ether, diisopropyl ether, tetrahydrofuran, dioxane, dimethoxyethane or diethylene glycol dimethyl ether, with halogenated hydrocarbons or aromatic hydrocarbons being used preferably, and dichloromethane, chloroform or toluene being used most preferably. Examples of the chlorinating agents used may include methanesulfonyl chloride, oxalyl chloride, thionyl chloride, phosphorous oxychloride, phosphorous trichloride, phosphorous pentachloride and hydrochloric acid, with thionyl chloride and hydrochloric acid being used preferably. Examples of bases used may include tertiary alkylamines such as trimethylamine or triethylamine, and pyridine, with triethylamine being used most preferably.
Although varying according to the starting raw materials, the solvent and the chlorinating agent, the reaction temperature is generally from -20 to 30°C and preferably from 0 to 10°C.
Although varying according to the starting raw materials, the solvent, the chlorinating agent and the reaction temperature, the reaction time is generally from 10 minutes to 6 hours and preferably from 10 minutes to 2 hours.
Furthermore, the compounds of the present step can be used as is in the next step without particularly having to be isolated.
In the case of brominating, a reagent such as bromine/red phosphorous, phosphorous tribromide or phosphorous pentabromide can be used, or in the case of iodinating, a reagent such as iodine/red phosphorous can be used. In addition, a bromide or iodide can be respectively obtained by allowing a reagent such as sodium bromide or sodium iodide to act on the leaving group synthesized in step A-1.

(Step A-2) Thioetherification

This step is a step for producing compound (4) by reacting compound (2) with compound (3a) or a salt thereof (and particularly a hydrochloride) in the absence of solvent or in an inert solvent and in the presence or absence of base.
There are no particular limitations on the solvent used provided it dissolves the starting raw materials to a certain extent and does not impair the reaction, examples of which may include alcohols such as methanol, ethanol, n-propanol, isopropanol, n-butanol, isobutanol, t-butanol, isoamyl alcohol, diethylene glycol, glycerin, octanol, cyclohexanol

or methyl cellosorb; halogenated hydrocarbons such as chloroform, dichloromethane, 1,2-dichloroethane or carbon tetrachloride; ethers such as diethyl ether, diisopropyl ether, tetrahydrofuran, dioxane, dimethoxyethane or diethylene glycol dimethyl ether; aromatic hydrocarbons such as benzene or toluene, N,N-dimethylformamide, dimethylsulfoxide, water and mixed solvents thereof, with dichloromethane, alcohols, ethers or mixed solvents of ethers and toluene being used preferably, and methanol, tetrahydrofuran or a mixed solvent of tetrahydrofuran and toluene being used most preferably.

Examples of bases used may include inorganic bases such as sodium hydride, potassium hydride, lithium carbonate, sodium carbonate, potassium carbonate, lithium hydroxide, sodium hydroxide or potassium hydroxide; and organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo [4.3.0]nona-5-ene (DBN) 1,4-diazabicyclo[2.2.2]octane (DABCO) or 1,8-diazabicyclo [5.4.0]undeca-7-ene (DBU), with inorganic bases such as sodium hydride, potassium hydride, lithium hydroxide, sodium hydroxide or potassium hydroxide or triethylamine being used preferably, and sodium hydroxide or triethylamine being used most preferably.

Although varying according to the starting raw materials, the solvent and the base, the reaction temperature is generally from 0 to 100°C and preferably from 10 to 50°C.
Although varying according to the starting raw materials, the solvent, the base and the reaction temperature, the reaction time is generally from 30 minutes to 3 days.

(Step A-3) Oxidation Reaction

This step is a step for producing compound (1) by reacting an oxidizing agent with compound (4) in the presence or absence of solvent.

There are no particular limitations on the solvent used provided it dissolves the starting raw materials to a certain extent and does not impair the reaction, examples of which may include alcohols such as methanol, ethanol, n-propanol, isopropanol, n-butanol, isobutanol, t-butanol, isoamyl alcohol, diethylene glycol, glycerin, octanol, cyclohexanol or methyl cellosorb; aromatic hydrocarbons such as benzene or toluene; halogenated hydrocarbons such as chloroform, dichloromethane, 1,2-dichloroethane or carbon tetrachloride; amides such as formamide, N,N-dimethylformamide, N, N-dimethylacetoamide or hexamethyl phosphoric triamide; and nitriles such as acetonitrile, with aromatic hydrocarbons, alcohols, halogenated hydrocarbons or mixed solvents thereof being used preferably, and toluene, a mixed solvent of toluene and methanol or dichloromethane being used most preferably.

Examples of the oxidizing agents used may include hydrogen peroxide, t-butylhydroperoxide, cumene hydroperoxide, sodium periodate, peracetic acid, perbenzoic acid, 3-chloroperbenzoic acid and urea-hydrogen peroxide addition compound ($(NH_2)_2CO\text{-}H_2O_2$), with 3-chloroperbenzoic acid or cumene hydroperoxide being used preferably.
Furthermore, asymmetric oxidation can be carried out using methods described in the following publications: WO 96/02535, WO 2001/83473, WO 2004/087702, WO 2004/052881, WO 2004/052882, Adv. Synth. Catal. 2005, 347, 19-31, Chem. Rev. 2003, 103, 3651-3705, Tetrahedron Lett. 2004, 45, 9249-9252, Angew. Chem. Int. Ed. 2004, 43, 4225-4228 or Tetrahedron Asymmetry 2003, 14, 407-410.

More specifically, asymmetric oxidation can be carried out by reacting compound (4) with an oxidizing agent in the presence of an asymmetry inducer or asymmetry-inducing catalyst.
A peroxide such as hydrogen peroxide, tert-butylhydroperoxide, urea hydroperoxide or cumene hydroperoxide can be used for the oxidizing agent, cumene peroxide is used in the case in which the asymmetry inducer or asymmetry-inducing catalyst contains titanium, zirconium or hafnium in particular, while hydrogen peroxide is used in the case of containing vanadium.

Although an excess of oxidizing agent is used based on compound (4), the amount of oxidizing agent used is preferably 1.01 to 10 molar equivalents. The amount of oxidizing agent used is generally 1.05 equivalents in the case the asymmetry inducer or asymmetry-inducing catalyst contains titanium, 1.2 equivalents in the case it contains zirconium or hafnium, and 1.1 equivalents in the case it contains vanadium.

Examples of the asymmetry inducer or asymmetry-inducing catalyst may include the following:

(1) optically active titanium complexes such as complexes of an optically active diol, titanium (IV) alkoxide and water or alcohol;
(2) optically active zirconium complexes such as complexes of an optically active diol and zirconium (IV) alkoxide (with or without water);
(3) optically active hafnium complexes such as complexes of an optically active diol and hafnium (IV) alkoxide;
(4) optically active vanadium complexes such as complexes of an optically active Schiff base and vanadyl acetylacetonate;
(5) optically active iron complexes such as complexes of an optically active Schiff base and iron (III) acetylacetonate;

(6) optically active manganese complexes of an optically active Schiff base and manganese (III) (such as a salen-manganese complex); and
(7) optically active tungsten complexes obtained from an optically active chincona alkaloid and tungsten (III).

Examples of the optically active diols may include the following:

(1) alkyl diols, for example, tartrate esters such as (+) or (-)-dimethyl tartrate, diethyl tartrate, diisopropyl tartrate or dibutyl tartrate; and tartaric acid amides such as tartaric acid tetramethyldiamide; and
(2) aromatic diols such as (R) or (S)-binaphthol.

Examples of the optically active Schiff base may include Schiff bases derived from substituted salicylic aldehydes such as (S)-(-)-2-(3,5-di-tert-butylsalicylideneamino)- 3,3-dimethyl-1-butanol or (1R,2S)-1-[2-hydroxy-3,5-di-tert-butylbenzylidene]amino] indan-2-ol, and salen-type Schiff bases.

In the case of carrying out asymmetric oxidation, a base can be added as necessary. There are no particular limitations on the base used provided it does not impair the reaction, examples of which may include inorganic bases and organic bases, with tertiary amines such as diisopropylethylamine or triethylamine being used preferably, and diisopropylethylamine being used most preferably. The amount of base is generally 0.1 to 1 equivalent based on compound (4).

Furthermore, a base is generally not used in the case of using an asymmetry inducer of asymmetry-inducing catalyst containing vanadium.

Examples of solvents used in the case of carrying out asymmetric oxidation may include aromatic hydrocarbons such as toluene, benzene or xylene; halogenated hydrocarbons such as dichloromethene or chloroform; and esters such as ethyl acetate, with toluene or tert-butyl methyl ether being used preferably in the case of using an asymmetry inducer or asymmetry-inducing catalyst containing titanium, zirconium or hafnium, and acetonitrile or dichloromethane being used preferably in the case of using an asymmetry inducer or asymmetry-inducing catalyst containing vanadium.

In addition, it is effective to add water in the case of using an asymmetry inducer or asymmetry-inducing catalyst containing titanium, and the amount of water added, including the moisture content of the solvent, reaction agents (excluding oxidizing agent) and base, is preferably 0.1 to 0.33 equivalents and most preferably 0.13 to 0.25 equivalents based on compound (4). In addition, a 3A molecular sieve can also be used to control moisture content.

Isopropanol is effective for the alcohol used in the case of synthesizing a complex of a titanium (IV) alkoxide and an alcohol, and 1.2 equivalents of isopropanol are generally used based on titanium.

Although varying according to the starting raw materials, the solvent and the oxidizing agent, the reaction temperature is generally from -100 to 100°C and preferably from -70 to 70°C.

Although varying according to the starting raw materials, the solvent, the oxidizing agent and the reaction temperature, the reaction time is generally from 15 minutes to 72 hours and preferably from 30 minutes to 24 hours.

In addition, a compound obtained in the manner described above can be converted to a salt in accordance with the ordinary methods. For example, a base is reacted with compound (1) in the presence or absence of solvent. Examples of solvents may include acetonitrile, alcohols such as methanol or ethanol, water and mixed solvents thereof, with a mixed solvent of ethanol and water being used preferably. Examples of the bases may include alkaline metal hydroxides such as lithium hydroxide, sodium hydroxide or potassium hydroxide; alkaline earth metal hydroxides such as magnesium hydroxide; and alkoxides such as sodium methoxide, sodium t-butoxide, sodium t-pentoxide or magnesium methoxide, with an aqueous solution of sodium hydroxide being used preferably. The reaction temperature is generally from -50 to 50°C and preferably from 10 to 40°C. The reaction time is generally from 1 minute to 2 hours and preferably from 1 minute to 1 hour.

An alkaline metal salt such as a sodium salt or potassium salt can be converted to a metal salt such as a barium salt, a magnesium salt or a zinc salt that reacts by a salt exchange reaction with a metal chloride or metal sulfate compound, such as barium chloride, magnesium chloride, magnesium sulfate or zinc sulfate, in the presence or absence of a solvent. Compound (1) can be obtained in the form of a metal salt by supplying compound (4) to a chlorination reaction without going through an isolation procedure following the oxidation reaction thereof.

Compound (2) and compound (3), which are intermediates in the above-mentioned Method A, can use commercially available products, or can be easily produced by a person with ordinary skill in the art in accordance with the ordinary methods from the commercially available products. In addition, compound (3) in particular can also be produced according to Method B described below.

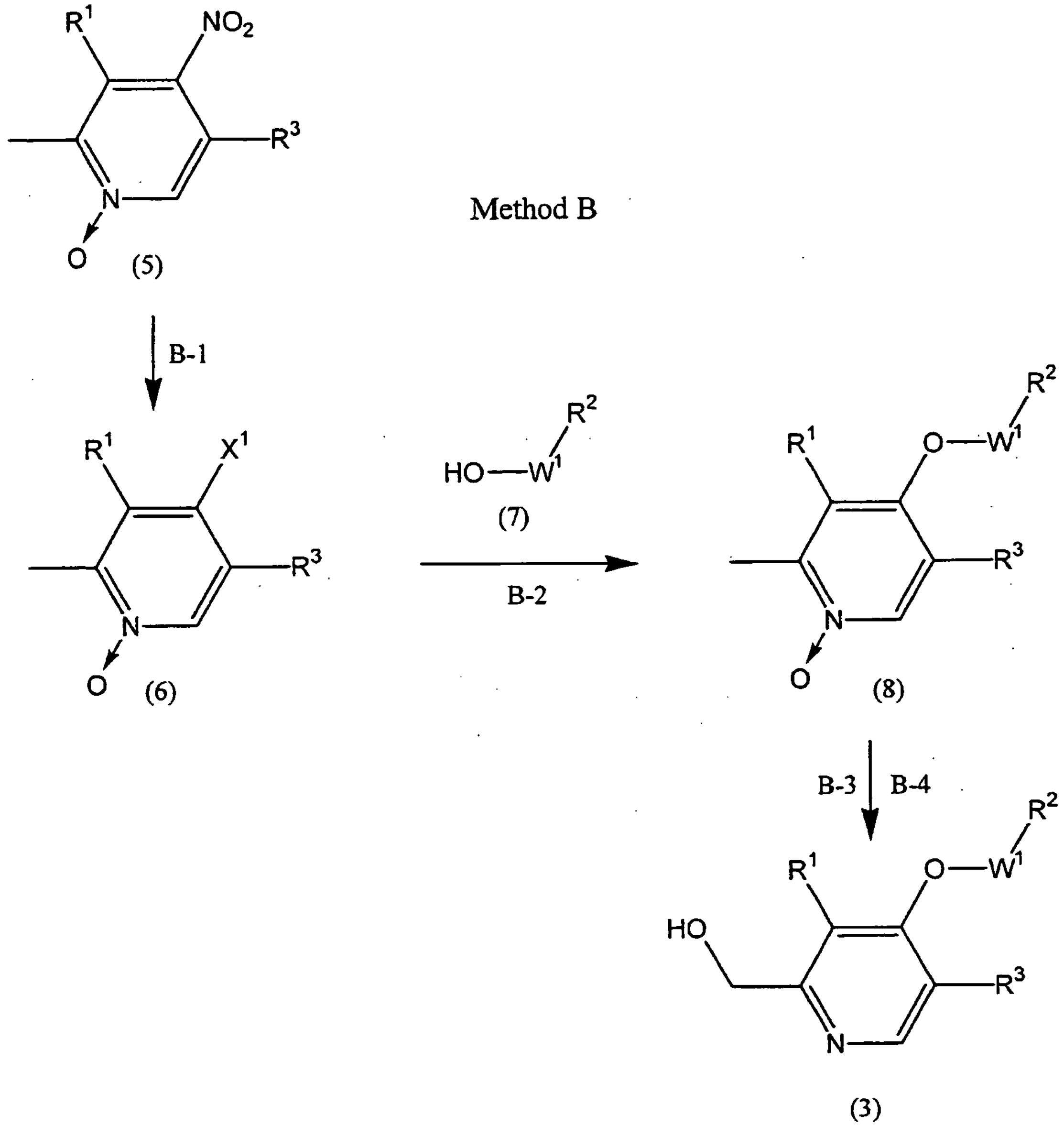


In the above formulas, R1, R2, R3 and W1 are the same as defined above, and X1 represents a halogen atom, preferably a chlorine atom, bromine atom or iodine atom, and more preferably a chlorine atom.

The following provides an explanation of each step of Method B.

Step (B-1) Halogenation Reaction (Using the Typical Example of Chlorination Reaction)

This step is a step for producing compound (6) by reacting a chlorinating agent with compound (5) in the absence of solvent or in an inert solvent.

In this step, although it is desirable to carry out the reaction in the presence of the chlorinating agent without using a solvent, in the case of using a solvent, there are no particular limitations on the solvent used provided it dissolves the starting raw materials to a certain extent and does not impair the reaction, examples of which may include halogenated hydrocarbons such as chloroform, dichloromethane, 1,2-dichloroethane or carbon tetrachloride; and ethers such as diethyl ether, diisopropyl ether, tetrahydrofuran, dioxane, dimethoxyethane or diethylene glycol dimethyl ether.

Examples of the chlorinating agents used may include acetyl chloride, oxalyl chloride, thionyl chloride, phosphorous oxychloride, phosphorous trichloride and phosphorous pentachloride, with acetyl chloride being used preferably.

Although varying according to the starting raw materials, the solvent and the chlorinating agent, the reaction temperature is generally from -50 to 30°C and generally from -30 to 10°C.

Although varying according to the starting raw materials, the solvent, the chlorinating agent and the reaction temperature, the reaction time is generally from 30 minutes to 8 hours and preferably from 1 to 5 hours.

In the case of brominating, a reagent such as acetyl bromide, hydrogen bromide, bromine/red phosphorous, phosphorous tribromide or phosphorous pentabromide is used, in the case of iodinating, a reagent such as iodine/red phosphorous can be used, or a reagent such as sodium iodide can be allowed to act following bromination.

(Step B-2) R2-W1-O Group Introduction Reaction

This step is a step for producing compound (8) by reacting compound (6) with alcohol (7) in the form of R2-W1-OH (wherein R2 and W1 are the same as defined above) in the absence of solvent or in an inert solvent and in the presence of base.

There are no particular limitations on the solvent used provided it dissolves the starting raw materials to a certain extent and does not impair the reaction, examples of which may include aliphatic hydrocarbons such as hexane, heptane, ligroin or petroleum ether; halogenated hydrocarbons such as chloroform, dichloromethane, 1,2-dichloroethane or carbon tetrachloride; aromatic hydrocarbons such as benzene or toluene; ethers such as diethyl ether, diisopropyl ether, tetrahydrofuran, dioxane, dimethoxyethane or diethylene glycol dimethyl ether; amides such as formamide, N,N-dimethylformamide, N,N-dimethylacetoamide, hexamethyl phosphoric triamide or N-methylpyrrolidone, dimethylsulfoxide, water and mixed solvents thereof, with dimethylsulfoxide, ethers or amides being used preferably, and dimethylsulfoxide being used most preferably.

Examples of bases used may include alkaline metal carbonates such as lithium carbonate, sodium carbonate or potassium carbonate; alkaline metal hydroxides such as lithium hydroxide, sodium hydroxide or potassium hydroxide; metal alkoxides such as lithium methoxide, sodium methoxide, sodium ethoxide or potassium t-butoxide; alkaline metal hydrides such as lithium hydride, sodium hydride or potassium hydride; alkaline metal alkoxides prepared from alkaline metals, n-butyl lithium and lithium diisopropylamide, with alkaline metal hydrides being used preferably, and sodium hydride being used most preferably.

Although varying according to the starting raw materials, the solvent and the base, the reaction temperature is generally from 0 to 100°C, preferably from 10 to 100°C in the case alcohol (7), namely R2-W1-OH, is a primary alcohol, and preferably from 50 to 100°C in the case it is a secondary alcohol.

Although varying according to the starting raw materials, the solvent, the base and the reaction temperature, the reaction time is generally from 15 minutes to 48 hours and preferably from 30 minutes to 12 hours.

(Step B-3) Rearrangement to Acetic Acid Ester

This step is a step for producing an acetic acid ester form of compound (3) by reacting acetic anhydride with compound (8) in the absence of solvent and in the presence or absence of base.

Examples of bases used may include tertiary alkylamines such as trimethylamine, diisopropylethylamine or triethylamine and pyridine, with triethylamine being used preferably.

Although varying according to the starting raw materials and the solvent, the reaction temperature is generally from 20 to 150°C, preferably from 20 to 60°C in the presence of base, and preferably from 50 to 100°C in the absence of base.

Although varying according to the starting raw materials, the solvent and the reaction temperature, the reaction time is generally from 10 minutes to 6 hours and preferably from 30 minutes to 5 hours.

The acetic anhydride is generally evaporated following the reaction, and the resulting residue can be used as is in the next step. In addition, compound (4) can also be obtained by carrying out step A-2 in the above-mentioned Method A starting with the acetic acid ester form.

(Step B-4) Hydrolysis Reaction

This step is a step for producing compound (3) by reacting a base with the compound obtained in step B-3 above in the presence or absence of solvent.

There are no particular limitations on the solvent used provided it dissolves the starting raw materials to a certain extent and does not impair the reaction, examples of which may include water, alcohols such as methanol, ethanol, n-propanol, isopropanol, n-butanol, isobutanol, t-butanol, isoamyl alcohol, diethylene glycol, glycerin, octanol, cyclohexanol or methyl cellosorb; aliphatic hydrocarbons such as hexane, heptane, ligroin or petroleum ether; ethers such as diethyl ether, diisopropyl ether, tetrahydrofuran, dioxane, dimethoxyethane or diethylene glycol dimethyl ether; halogenated hydrocarbons such as chloroform, dichloromethane, 1,2-dichloroethane or carbon tetrachloride; amides such as formamide, N,N-dimethylformamide, N,N-dimethylacetoamide or hexamethyl phosphoric triamide, and mixed solvents thereof, with alcohols or mixed solvents of alcohol and water being used preferably, and a mixed solvent of

methanol and water being used most preferably.

Examples of bases used may include alkaline metal carbonates such as lithium carbonate, sodium carbonate or potassium carbonate; alkaline metal hydroxides such as lithium hydroxide, sodium hydroxide or potassium hydroxide; metal alkoxides such as lithium methoxide, sodium methoxide, sodium ethoxide or potassium t-butoxide; and ammonias such as aqueous ammonia or concentrated ammonia-methanol, with alkaline metal hydroxides being used preferably, and sodium hydroxide being used most preferably.

Although varying according to the starting raw materials, the solvent and the base, the reaction temperature is generally from 0 to 60°C and preferably from 10 to 40°C.

Although varying according to the starting raw materials, the solvent, the base and the reaction temperature, the reaction time is generally from 10 minutes 6 hours.

The following provides an explanation of the formation of a sulfenamide form from compound (1) according to the present invention (refer to Method T below).

# Method T

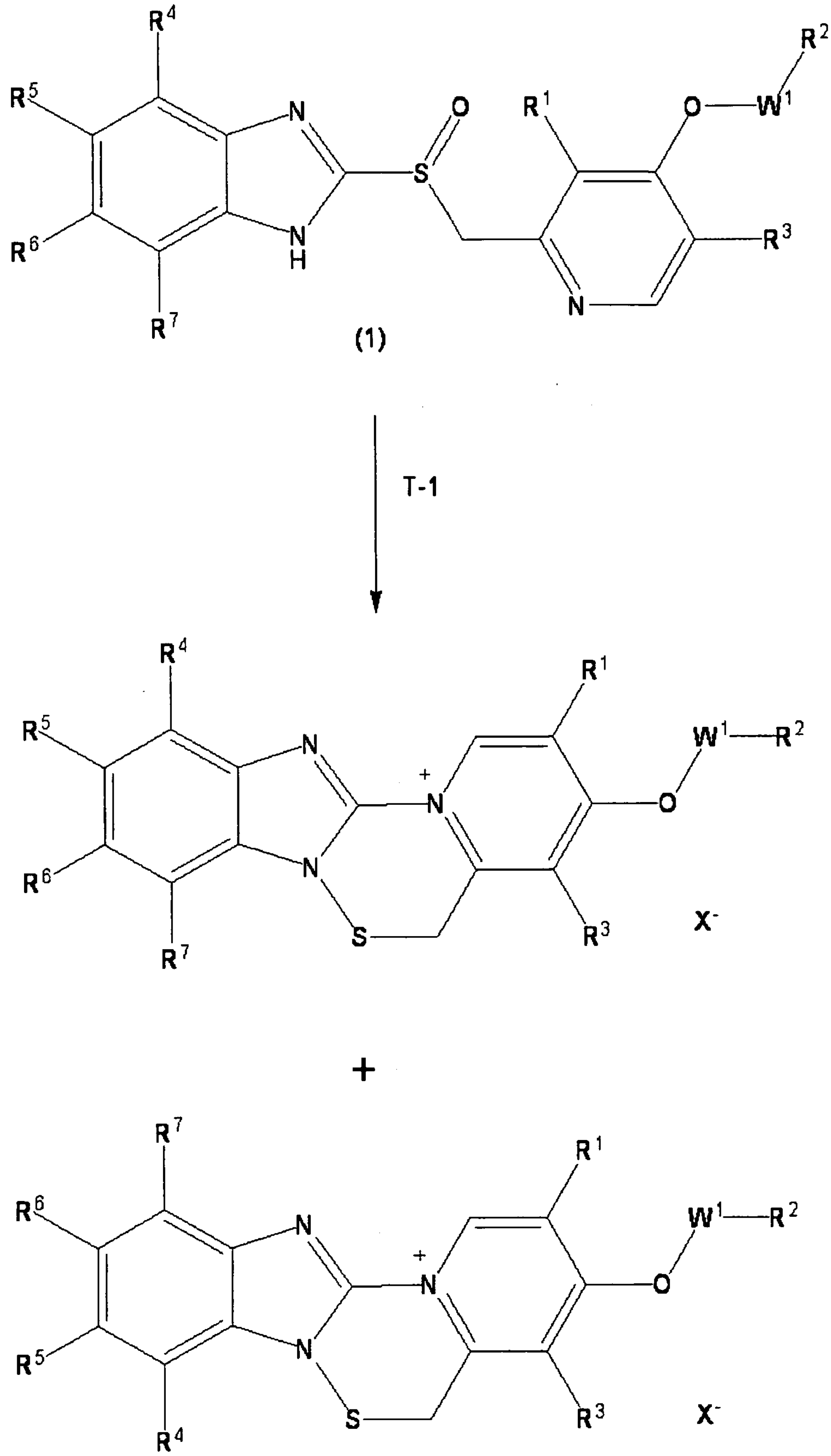


(1)

(2a)

+

(2b)

In the above formulas, R1, R2, R3, R4, R5, R6, R7 and W1 are the same as defined above, and X- represents $Cl^-$, $Br^-$, $I^-$, $BF_4^-$, $PF_6^-$, $HSO_4^-$, $SO_4^{2-}$, $CH_3SO_3^-$, 4-Me-Ph-$SO_3^-$, $PO_4^{3-}$, $ClO_4^-$ or $AuCl_4^-$.

This step is a step for producing compound (2a) and compound (2b) by reacting compound (1) in the presence or absence of solvent and in the presence of acid. Compound (2a) and compound (2b) can be produced at nearly the same ratio or with one or the other at a high ratio according to the reaction conditions or the influences of substituents.

There are no particular limitations on the solvent used provided it dissolves the starting raw materials to a certain extent and does not impair the reaction, examples of which may include ethers such as ether and tetrahydrofuran; alcohols such as methyl alcohol or ethyl alcohol; water, chloroform, dichloromethane, acetone, acetonitrile, dimethylformamide, dimethylsulfoxide and mixture thereof.

The target compounds (2a) and (2b) can be obtained by heating and reacting compound (1) in the form of a pyridine derivative in the presence of acid in accordance with the ordinary methods.

Examples of acids used may include tetrafluoroboric acid, hexafluorophosphoric acid, sulfuric acid, hydrochloric acid, hydrobromic acid, hydroiodic acid, perchloric acid, methanesulfonic acid and toluenesulfonic acid. The acid is preferably used at 1 to 3 equivalents based on compound (1).

Although the reaction temperature is from -40 to about 100°C, it is preferably from about 30 to 50°C. The reaction time is from several minutes to several hours.

The compound (1) used as the starting substance in the present reaction can be produced by the method as indicated in the previously described Method A or Method B.

After completing the reactions of each step in each of the above-mentioned methods, the target compound of each step can be collected from the reaction mixture in accordance with the ordinary methods.

For example, in the case the overall reaction mixture is a liquid, the reaction mixture is returned to room temperature or cooled with ice as desired and suitably acid, base, oxidizing agent or reducing agent is neutralized, followed by the addition of an organic solvent such as water and ethyl a acetate that is not miscible and does not react with the target compound and separation of the layer containing the target compound. Next, a solvent that is not miscible with the resulting layer and does not react with the target compound is added followed by washing the layer containing the target compound and separating the layer. In addition, the target compound can be collected, if the layer is an organic layer, by drying using a drying agent such as potassium carbonate, anhydrous magnesium sulfate or anhydrous sodium sulfate followed by evaporating the solvent. In addition, if the layer is an aqueous layer, the target compound can be collected by freeze-drying after electrically desalting.

In addition, in the case the overall reaction mixture is a liquid and when possible, the target compound can be collected by evaporating substances other than the target compound (such as solvents and reagents) at a normal pressure or under a reduced pressure.

Moreover, in the case only the target compound precipitates as a solid or in the case the overall reaction mixture is a liquid and only the target compound has precipitated as a solid during the collection process, the target compound can be further collected by first filtering out the target compound by a filtration method, washing the filtered target compound with a suitable organic or inorganic solvent, drying and then treating the mother liquor in the same manner as the case of the overall reaction mixture being a liquid as described above.

Moreover, in the case a reagent or catalyst is only present as a solid or the overall reaction mixture is a liquid, only the reagent or catalyst has precipitated as a solid during the collection process, and the target compound is dissolved in solution, the target compound can be collected by first filtering out the reagent or catalyst by a filtration method, washing the filtered reagent or catalyst with an organic or inorganic solvent, combining the resulting washing with the mother liquid and then treating the resulting mixture in the same manner as the case of the overall reaction mixture being a liquid.

Particularly in the case substances other than the target compound contained in the reaction mixture do not impair a reaction of a subsequent step, the reaction mixture can be used as in the subsequent step without isolating the target compound.

Recrystallization methods, various types of chromatographic methods or distillation methods can be suitably carried out to improve the purity of the target compound collected according to the methods described above.

In the case the collected target compound is a solid, the purity of the target compound can generally be improved by recrystallization. A single solvent or a mixture of a plurality of solvents that do not react with the target compound can be used for recrystallization. More specifically, the target compound is first dissolved in a single solvent or a plurality of solvents that do not react with the target compound either at room temperature or while heating. The target compound can then be crystallized from the solution by cooling the resulting solution with ice water and the like, stirring or allowing to stand at room temperature, or adding a solvent in which the solubility of the target compound is low.

The purity of the target compound can also be improved by various types of chromatography. In general, a weakly acidic silica gel can be used, examples of which may include Silica Gel 60 (70 to 230 mesh or 340 to 400 mesh) manufactured by Merck & Co., Inc., BW-300 (300 mesh) manufactured by Fuji Silysia Chemical Ltd. and Disposable Intermediate Pressure Isolating Packed Column (High Flush) manufactured by Yamazen Corp. In the case, for example,

the target compound is basic and adsorption with the silica gels described above is excessively strong, an NH silica gel can also be used, examples of which may include the Propylamine Coating Silica Gel (200 to 350 mesh) manufactured by Fuji Silysia Chemical Ltd. and the Disposable Intermediate Pressure Isolating Packed Column (High Flush Amine) manufactured by Yamazen Corp. In addition, in the case the target compound is bipolar or requires elution with a highly polar solvent such as methanol, NAM-200H or NAM-300H manufactured by Nam Laboratories Ltd. can be used. A target compound having improved purity can be obtained by using these silica gels to elute the target compound with a single or mixed solvent that does not react with the target compound followed by evaporating the solvent.

In the case the collected target compound is a liquid, the purity of the target compound can be improved by distillation. In distillation, the target compound can be distilled at a normal pressure or under a reduced pressure at room temperature or while heating.

Although the above has indicated typical examples of methods for producing compound (1a), compound (1b) or a salt thereof according to the present invention, a salt or a solvate such as a hydrate may also be formed, each differs according to the starting raw materials, solvent used and the like, and there are no particular limitations thereon provided they do not impair the reaction. The solvent used also varies according to the starting raw materials, the reagents and the like, and it goes without saying that there are no particular limitations thereon provided it dissolves the starting raw materials to a certain extent without impairing the reaction.

In the case the compound (1b) according to the present invention is obtained in a free form, the compound (1b) can be converted to a salt that may be formed by the compound (1b) or to the state of a solvate thereof in accordance with the ordinary methods.

In the case a compound according to the present invention is used as medicament, the compound according to the present invention is generally mixed with a suitable additive and used in the form of a preparation. However, this does not negate the direct use of the compound according to the present invention as a pharmaceutical in an unmodified form.

Examples of the above-mentioned additives may include vehicles, binders, lubricants, disintegration agents, colorants, flavoring agents, emulsifiers, surfactants, solubilizers, suspending agents, isotonizing agents, buffers, antiseptics, antioxidants, stabilizers and absorption enhancers commonly used in pharmaceuticals, and these can be used in suitable combinations as desired.

The compound (1 a) and the compound (1 b) according to the present invention can be produced according to the methods described in the following examples, and the effects of these compounds can be confirmed by the methods described in the following test examples. However, these examples are intended only to be exemplary, and the present invention is not limited to these specific examples in any case.

Examples

4-Chloro-2,3-dimethylpyridine 1-oxide (Sanyo Fine Co., Ltd., J. Med. Chem., 1998, 41, 1777-1788)
Sodium hydride, in oil (Wako Pure Chemical Industries Ltd.)
Acetic anhydride (Kanto.Chemical Co., Inc.)
5N aqueous sodium hydroxide (Wako Pure Chemical Industries Ltd.)
Methanesulfonyl chloride (Tokyo Kasei Kogyo Co., Ltd.)
Triethylamine (Kanto Chemical Co., Inc.)
2-Mercaptobenzimidazole (Tokyo Kasei Kogyo Co., Ltd.)
2 N hydrochloric acid (Wako Pure Chemical Industries Ltd.)
Iron (III) chloride (Kanto Chemical Co., Inc.)
Cyclobutanone (Avocado Corp.)
3-Chloroperbenzoic acid (Tokyo Kasei Kogyo Co., Ltd.)
1 N aqueous sodium hydroxide (Wako Pure Chemical Industries Ltd.)
1,2,4-Butanetriol (Tokyo Kasei Kogyo Co., Ltd.)
p-Toluenesulfonic acid monohydrate (Tokyo Kasei Kogyo Co., Ltd.)
Acetone (Wako Pure Chemical Industries Ltd.)
Cyclopentanone (Tokyo Kasei Kogyo Co., Ltd.)
Cyclohexanone (Tokyo Kasei Kogyo Co., Ltd.)
Tetrahydro-4*H*-pyran-4-one (Tokyo Kasei Kogyo Co., Ltd.)
3-Pentanone (Tokyo Kasei Kogyo Co., Ltd.)
Hexafluorophosphoric acid (Aldrich Corp.)

Furthermore, an atom indicated with an asterisk (*) in the chemical structural formulas in the examples refers to an asymmetric atom.

(Production Example 1) (2,2-Dimethyl-1,3-dioxan-5-yl) methanol

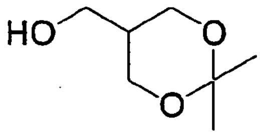

A mixture of 2-(hydroxymethyl)-1,3-propanediol (4.09 g, 38.5 mmol), acetone (130 ml, 1.768 mmol) and 70% perchloric acid (1.37 g, 9.55 mmol) was stirred for 21 hours at room temperature. The reaction mixture was concentrated after adjusting to pH 9 with concentrated ammonia. The residue was purified by silica gel column chromatography (silica gel: 100 g, elution solvent: heptane, heptane/ethyl acetate = 1/3) to obtain the target compound (4.83 g, yield: 85.8%) as a colorless oily substance.

$^1$H-NMR (400 MHz, DMSO-$d_6$) δppm: 1.29 (3H,s), 1.30 (3H,s), 1.64-1.74 (1H,m), 3.35-3.41 (2H,m), 3.61 (2H,dd,J=7,12 Hz), 3.82 (2H,dd,J=4,12 Hz), 4.54 (1H,t,J=5 Hz)

(Production Example 2) 2,3,5-Trimethylpyridine 1-oxide

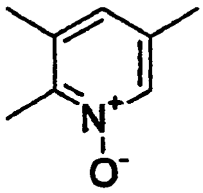

2,3,5-Trimethylpyridine (1.43 kg, 11.80 mol) were added to acetic acid (1.43 kg, 23.83 mol) over 15 minutes. After 15 minutes, 35% aqueous hydrogen peroxide (1.38 kg, 14.2 mol) was added dropwise over 30 minutes followed by stirring overnight at 90 to 95°C. Sodium sulfite (220 g) was added to the reaction liquid. The reaction mixture was added to a mixture of sodium carbonate (2.5 kg) and water (12 L) followed by extraction with chloroform (3.0 L x 4). The resulting organic layer was concentrated until crystals precipitated followed by the addition of n-hexane (2.5 L) to the precipitate and stirring overnight while cooling with ice. The resulting crystals were filtered to obtain 1.53 kg of the target substance.

(Production Example 3) 4-Nitro-2,3,5-trimethylpyridine 1-oxide

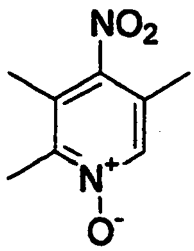

4-Nitro-2,3,5-trimethylpyridine 1-oxide (1.38 kg, 10.1 mol) was added to 98% sulfuric acid (4.93 kg, 49.3 mol). 97% nitric acid (1.44 kg) was added dropwise over 50 minutes followed by heating at 85°C for 4 hours. The reaction liquid was then added to a mixture of ammonium bicarbonate (10.6 kg) and water (9.0 L) followed by extraction with ethyl acetate (3.0 L x 3). The resulting organic layer was concentrated and vacuum-dried overnight to obtain 1.50 kg of the target substance.

(Production Example 4) 4-Chloro-2,3,5-trimethylpyridine 1-oxide

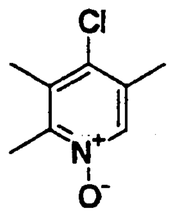

To 4-nitro-2,3,5-trimethylpyridine 1-oxide (850 g, 4.67 mol) were added water (400 g) and 36% concentrated hydrochloric acid (1.69 kg), which was heated to 70°C. N,N-Dimethylformamide (115 mL) was added thereto followed by heating to 100°C. Following completion of the reaction, the mixture was cooled to 20°C followed by the addition of potassium carbonate (1.40 kg) and water (7 L) thereto, extraction with chloroform, drying over sodium sulfate and concentrating. The resulting crude form was stirred for 2 hours in a mixture of diisopropyl ether (500 mL) and n-hexane (1.0 L) followed by suction filtration. The resulting wet form was vacuum-dried overnight to obtain 666.4 g of the target substance.

(Production Example 5) 4-(2,2-Dimethyl-1,3-dioxan-5-ylmethoxy)-2,3,5-trimethylpyridine 1-oxide

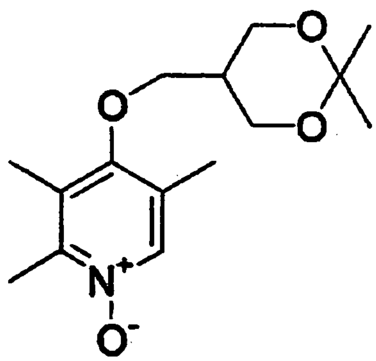

A mixture of 4-nitro-2,3,5-trimethylpyridine 1-oxide (840 g), (2,2-dimethyl-1,3-dioxane-5-yl) methanol (688 g) and toluene (2.52 L) was refluxed while removing water. Potassium hydroxide (0.58 kg) was added thereto over 3 hours 45 minutes while continuing azeotropic dehydration followed by further continuing azeotropic dehydration for 2.5 hours. The reaction mixture was cooled to 30°C or lower followed by the addition of ethyl acetate (2.5 L) and 17% brine (3.5 L) and allowing to stand undisturbed overnight. The ethyl acetate layer was isolated and the aqueous layer was extracted with ethyl acetate (1.0 L x 3). The combined ethyl acetate layer was filtered through Celite followed by concentrating under a reduced pressure to obtain 1.20 kg of the target substance.

(Production Example 6) [4-(2,2-Dimethyl-1,3-dioxan-5-yl)methoxy-3,5-dimethylpyridin-2-yl] methanol monohydrate

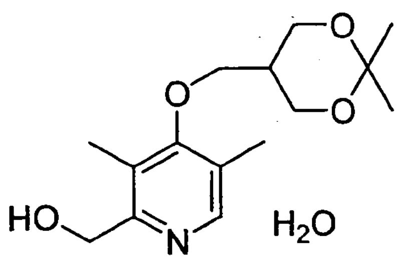

Acetic anhydride (1.10 kg) was added dropwise to a mixture of 4-(2,2-dimethyl-1,3-dioxan-5-yl)methoxy-2,3,5-trimethylpyridine 1-oxide (1.20 kg) and sodium acetate (0.18 kg), which were heated to 50 to 60°C over 1.5 hours. After

0.5 hours had elapsed, the mixture was heated for 4.5 hours at 80°C and then allowed to stand after cooling to an internal temperature of 30°C or lower followed by concentrating under a reduced pressure. The resulting residue was dissolved in methanol (1.0 L) followed by the addition of a mixture of 48% aqueous sodium hydroxide (0.71 kg) and ice water (2.85 L) over 1 hour. After stirring for 5 hours 45 minutes at room temperature, the mixture was concentrated under a reduced pressure. Water (3.0 L) was added to the concentrated residue followed by extraction with toluene (2.3 L x 4) and washing the combined toluene layer with water (1.2 L). The resulting organic layer was filtered through Celite and then concentrated. Diisopropyl ether (1.15 L) was added to the resulting residue at room temperature followed by the further addition of hot water (45°C, 74 mL). Following confirmation of crystal precipitation, the mixture was stirred for 1 hour at 25°C followed by the addition of heptane (3.6 L) and continuing to stir overnight. Moreover, after stirring for 5 hours while cooling with ice, the mixture was filtered to obtain yellow crystals. Diisopropyl ether (3.5 L) was added to the resulting yellow crystals followed by dissolving at 50°C. After removing impurities by filtration, the mixture was cooled and aged overnight at 5°C. The resulting crystals were filtered, washed with heptane (0.5 L) and dried to obtain 0.69 kg of the target substance.

(Production Example 7) 2-[[[4-(2,2-Dimethyl-1,3-dioxan-5-yl)methoxy-3,5-dimethylpyridin-2-yl]methyl]thio]-1*H*-benzimidazole

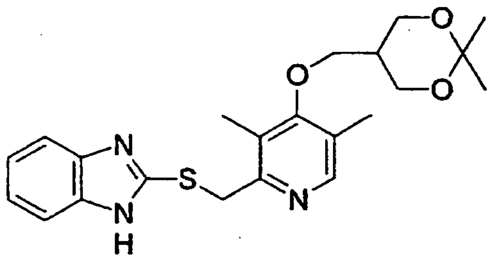

Azeotropic dehydration was carried out by adding [4-(2,2-dimethyl-1,3-dioxan-5-yl)methoxy-3,5-dimethylpyridin-2-yl] methanol monohydrate (690 g) to toluene (2.1 L x 5, 1.75 L x 1). Toluene (393 mL) was added to the resulting concentrate to obtain 921 g of a toluene solution of [4-(2,2-dimethyl-1,3-dioxan-5-yl) methoxy-3,5-dimethyl-2-yl] methanol.

A toluene solution of [4-(2,2-dimethyl-1,3-dioxan-5-yl)methoxy-3,5-dimethylpyridin-2-yl] methanol (845.7 g, content: 61.7%, contained amount: 521.8 g, 1.855 mol), tetrahydrofuran (2609 mL), toluene (669 mL) and triethylamine (375.3 g, 3.709 mol) were sequentially added thereto under a nitrogen atmosphere followed by stirring while cooling with dry ice/ethanol. Methanesulfonyl chloride (254.9 g, 2.226 mol) were added dropwise over 42 minutes 30 minutes after the start of cooling. Following completion of addition, the mixture was stirred while cooling on an ice water bath. Approximately 1.5 hours later, a tetrahydrofuran solution (3653 mL) of 2-mercaptobenzimidazole (334.28 g, 2.226 mol) was added thereto over 2 minutes followed by continuing to stir for about 18 hours at room temperature.

After adding toluene (3653 mL) to the reaction liquid, 20% w/w aqueous sodium hydroxide solution (1852.4 g) was added followed by the further addition of $H_2O$ (2322 mL), extraction and separation. The organic layer was washed twice with 20% w/w aqueous ammonium chloride solution (4174 g) and further washed with $H_2O$ (4147 mL)

The resulting organic layer was concentrated under a reduced pressure (40°C) to obtain a brownish oily substance (2.40 kg, toluene content: 144.6 mL, tetrahydrofuran content: 168 mL, calculated from $^1$H-NMR spectrum).

The resulting brownish oily substance was transferred to a crystallization vessel, washed with toluene (119 mL) and stirred at room temperature. Ten minutes later, tert-butyl methyl ether (134 mL) was added thereto followed by continuing to stir at room temperature. Twenty minutes later, tert-butyl methyl ether (127 mL) was further added thereto followed by continuing to stir at room temperature. Thirty minutes later, tert-butyl methyl ether (266 mL) was added dropwise over 20 minutes followed by continuing to stir at room temperature. When tert-butyl methyl ether (522 mL) was further added dropwise thereto one minute later, crystal precipitation was confirmed 8 minutes later and addition was completed at a total elapsed time of 1 hour 20 minutes. After stirring at room temperature for 40 minutes, heptane (2348 mL) was added dropwise thereto over 1 hour 17 minutes followed by stirring overnight at room temperature.

About 15.5 hours after the heptane was added dropwise, the precipitated crystals were suction-filtered and rinsed with toluene/tert-butyl methyl ether/heptane (587 mL/391 mL/587 mL) followed by vacuum drying. The resulting wet crystals were blow dried (50°C) to obtain the target substance.

Yield: 619.0 g, content: 96.5%, contained amount: 597.3 g, yield percentage: 77.8% (based on contained amount), HPLC purity: 98.0%

<HPLC Analysis Conditions (reaction check, HPLC purity measurement and quantification)>

Column: YMC-Pack Pro C18AS-302 (5 μm, 4.6 mm x 150 mm I.D.)
Eluent: Solution A (MeCN/20 mM $AcONH_4$ aq. = 100/900 (v/v)), Solution B (MeCN/20 mM $AcONH_4$ aq. = 800/200 (v/v))
Flow rate: 1.0 mL/min
Detection: UV 254 nm
Oven temp.: 25°C
Sample temp.: 25°C
Gradient condition (time/solution B conc.): 0.01 min/0% → 25 min/100% → 30 min/100% → 30.01 min/0% → 40 min/stop
RT = 18.4 min

(Example 1) Sodium Salt of Optical Isomer Having Short Retention Time of Crude 2-[[[4-(2,2-dimethyl-1,3-dioxan-5-yl)methoxy-3,5-dimethylpyridin-2-yl]methyl]sulfinyl]-1 H-benzimidazole

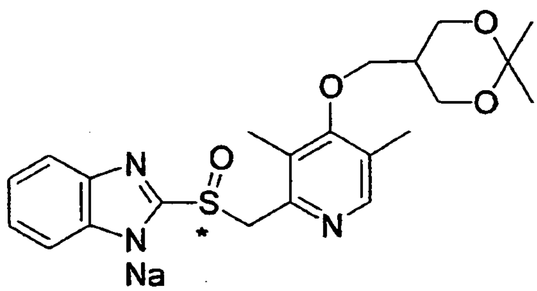

2-[[[4-(2,2-dimethyl-1,3-dioxan-5-yl)methoxy-3,5-dimethylpyridin-2-yl]methyl] thio]-1H-benzimidazole (580.3 g, content: 96.5%, contained amount: 560.0 g, 1.354 mol), toluene (3864 mL) and $H_2O$ (2.81 g, 0.156 mol) were sequentially added under a nitrogen atmosphere, which was stirred while heating at 60°C. Six minutes later, L-(+)-diethyl tartrate (122.9 g, 0.596 mol) were added to this suspension and washed with toluene (560 mL). Dissolution was confirmed 30 minutes later. Eight minutes later, titanium (IV) tetraisopropoxide (77.0 g, 0.271 mol) were added thereto and washed with toluene (56 mL) followed by stirring while heating for about one hour at the same temperature. After changing to cooling at 8°C, N,N-diisopropylethylamine (56.01 g, 0.742 mol) were added thereto and washed with toluene (280 mL). Ten minutes later, a toluene solution (840 mL) of cumene hydroperoxide (259.2 g, 1.422 mol) was added dropwise thereto over 47 minutes followed by stirring for about 18.5 hours at 8°C. Cooled 30% w/w aqueous sodium thiosulfate solution (2240 g) was added thereto followed by stirring for 12 minutes and discarding the aqueous layer. 4% w/w aqueous sodium hydroxide solution (2240 g) was added to the organic layer, stirred and then allowed to stand undisturbed followed by removing the aqueous layer to obtain an sodium hydroxide aqueous extract of an optical isomer having a short retention time of 2-[[[4-(2,2-dimethyl-1,3-dioxan-5-yl)methoxy-3,5-dimethylpyridin-2-yl]methyl]sulfinyl]-1*H*-benzimidazole as a brownish-yellow suspension. The sodium hydroxide aqueous extract of an optical isomer having a short retention time of 2-[[[4-(2,2-dimethyl-1,3-dioxan-5-yl)methoxy-3,5-dimethylpyridin-2-yl]methyl]sulfinyl]-1*H*-benzimidazole (2.98 kg) was added to toluene (7840 mL) and stirred. 20% w/w aqueous acetic acid solution (400 mL), 8% aqueous NaOH solution (50 mL) and 20% w/w aqueous acetic acid solution (8 mL) were sequentially added to this mixture followed by adjusting to pH 8.64, allowing to stand undisturbed, separating and discarding the aqueous layer. The organic layer was washed with 5% w/w aqueous saltwater solution (2240 g) and then separated to obtain a toluene extract (7.31 kg) of an optical isomer having a short retention time of 2-[[[4-(2,2-dimethyl-1,3-dioxan-5-yl)methoxy-3,5-dimethylpyridin-2-yl]methyl]sulfinyl]-1*H*-benzimidazole (contained amount of optical isomer having short retention time of 2-[[[4-(2,2-dimethyl-1,3-dioxan-5-yl)methoxy-3,5-dimethylpyridin-2-yl]methyl]sulfinyl]-1H-benzimidazole: 567.7 g, 1.322 mol) as a brownish-yellow solution.
A methanol solution (245.6 g, 1.286 mol) of 28.3% sodium methoxide was added to the resulting toluene extract over one minute while stirring at room temperature. Next, tert-butyl methyl ether (1120 mL) was added dropwise into this solution over 3 minutes followed by stirring at room temperature, confirming precipitation of crystals about 6 minutes later, and then continuing to stir as is for about 30 minutes. Moreover, tert-butyl methyl ether (7840 mL) was added dropwise thereto over 2 hours 40 minutes followed by continuing to stir overnight at room temperature.
About 13 hours after adding tert-butyl methyl ether dropwise, the precipitated crystals were suction-filtered and rinsed with toluene/tert-butyl methyl ether (1047 mL/1193 mL) followed by suction-drying for 15 minutes. The resulting wet crystals were then vacuum-dried (40°C) to obtain the target substance.

Yield: 546.8 g, content: 101.7%, amount contained: 546.8 g (based on content of 100%), yield: 90.9% (based on amount of yield), HPLC purity: 98.2%, enantiomeric excess: 100% ee

<HPLC Analysis Conditions (reaction check, HPLC purity measurement and quantification)>

Column: YMC-Pack Pro C18 AS-302 (5 μm, 4.6 mm x 150 mm I.D.)
Eluent: Solution A (MeCN/20 mM $AcONH_4$ aq. = 100/900 (vlv)), Solution B ($MeCN/20$ mM $AcONH_4$ aq. = 800/200 (v/v))
Flow rate: 1.0 mL/min
Detection: UV 254 nm
Oven temp.: 25°C
Sample temp.: 25°C
Gradient condition (time/solution B conc.): 0.01 min/0% → 25 min/100% → 30 min/100% → 30.01 min/0% → 40 min/stop
RT = 14.1 min

<HPLC Analysis Conditions (enantiomeric excess)>

Column: Daicel CHIRALPAK 1A (4.6 mm x 250 mm I.D.)
Eluent: EtOH/MTBE = 150/850 (v/v)
Flow rate: 1.0 mL/min
Detection: UV 284 nm
Oven temp.: 25°C
Sample temp.: 25°C

(Example 2) Sodium Salt of Optical Isomer Having Short Retention Time of Crude 2-[[[4-(2,2-dimethyl-1,3-dioxan-5-yl)methoxy-3,5-dimethylpyridin-2-yl]methyl]sulfinyl]-1 H-benzimidazole

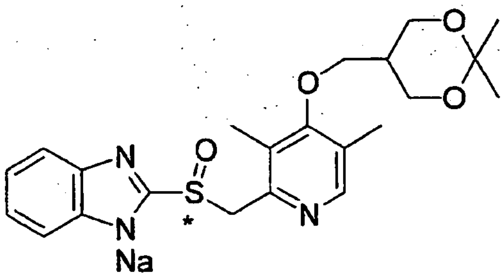

Sodium salt of an optical isomer having a short retention time of crude 2-[[[4-(2,2-dimethyl-1,3-dioxan-5-yl) methoxy-3,5-dimethylpyridin-2-yl]methyl]sulfinyl]-1H-benzimidazole (536.8 g, 1.189 mol) was added to ethanol (1074 mL) and stirred at room temperature followed by the addition of tert-butyl methyl ether (1074 mL). The resulting solution was suction-filtered with a Hyflo Super-Cel bed [107.4 g, sequentially washed with ethanol/tert-butyl methyl ether (1074 mL/1074 mL) and tert-butyl methyl ether (537 mL)] and then rinsed with ethanol/tert-butyl methyl ether (215 mU215 mL). The resulting filtrate was transferred to a crystallization vessel and washed with ethanol/tert-butyl methyl ether (54 mL/ 54 mL) followed by initiating stirring at room temperature. Tert-butyl methyl ether (1610 mL) was added dropwise thereto over 6 minutes followed by continuing to stir at room temperature. Eleven minutes later, tert-butyl methyl ether (268 mL) was added dropwise thereto over 2 minutes followed by continuing to stir and confirming crystal precipitation one minute later. After stirring as is for 31 minutes at room temperature, tert-butyl methyl ether (268 mL) was added dropwise thereto over 9 minutes. After stirring for 8 minutes at room temperature, tert-butyl methyl ether (8589 mL) was further added dropwise thereto over 1 hour 10 minutes followed by continuing to stir at room temperature.
About 22 hours after completion of addition of tert-butyl methyl ether, the precipitated crystals were suction-filtered while blowing in nitrogen followed by sequentially washing with ethanol/tert-butyl methyl ether (107 mU966 mL) and tert-butyl methyl ether (1074 mL) and suction-drying for 8 minutes. 531.10 g of the resulting wet crystals (584.54 g) were vacuum-dried (50°C) to obtain the target substance.
Yield: 419.6 g, HPLC purity: 99.4%

<HPLC Analysis Conditions (HPLC purity measurement and quantification)>

Column: YMC-Pack Pro C18 AS-302 (5 μm, 4.6 mm x 150 mm I.D.)
Eluent: Solution A (MeCN/20 mM $AcONH_4$ aq. = 100/900 (v/v)), Solution B (MeCN/20 mM $AcONH_4$ aq. = 800/200 (v/v))
Flow rate: 1.0 mL/min
Detection: UV 254 nm
Oven temp.: 25°C
Sample temp.: 25°C
Gradient condition (time/solution B conc.): 0.01 min/0% → 25 min/100% → 30 min/100% → 30.01 min/0% → 40 min/stop
RT = 14.1 min

(Example 3) 2-[[[4-[(2,2-Dimethyl-1,3-dioxan-5-yl)methoxy]-3,5-dimethylpyridin-2-yl]methyl]sulfinyl]-1*H*-benzimidazole (racemic form)

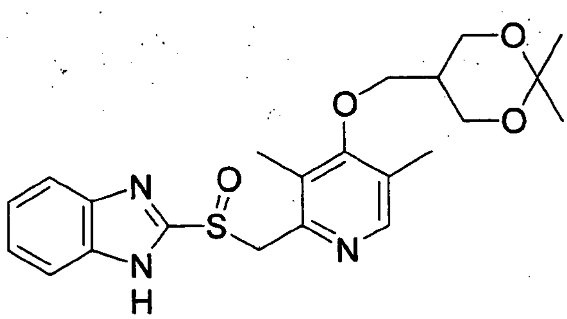

To a toluene (20 mL)-methanol (2 mL) solution of 2-[[[4-(2,2-dimethyl-1,3-dioxan-5-yl)methoxy]-3,5-dimethylpyridin-2-yl]methyl]thio]-1*H*-benzimidazole (424 mg, 1.03 mmol) was added a toluene (1 mL) - methanol (1 mL) solution of 3-chloroperbenzoic acid (246 mg, 0.927 mol for a content of 65%) at -65°C over 5 minutes under a nitrogen atmosphere followed by stirring the mixture for 45 minutes under the same conditions. Saturated aqueous sodium bicarbonate solution was then added to the reaction mixture. The mixture was then extracted with ethyl acetate. The organic layer was dried over anhydrous sodium sulfate followed by filtering and concentrating. The residue was purified by silica gel column chromatography (NH silica gel: 20 g, elution solvent: dichloromethane, dichloromethane/methanol = 10/1). Fractions containing the target compound were collected using ethyl acetate and concentrated. Diethyl ether was then added to the residue. The resulting precipitate was then filtered and washed with diethyl ether to obtain the target compound (274 mg, yield: 61.9%) as a white solid.
$^1$H-NMR (400 MHz, DMSO-$d_6$) $\delta_{ppm}$: 1.32 (3H,s), 1.36 (3H,s), 2.02-2.13 (1H,m), 2.16 (3H,s), 2.20 (3H,s), 3.74-3.84 (4H, m), 4.00 (2H,dd,J=4,12 Hz), 4.70 (1H,d,J=14 Hz), 4.79 (1 H,d,J=14 Hz), 7.26-7.33 (2H,m), 7.60-7.70 (2H,m), 8.18 (1 H,s)

(Example 4) 2-[[[4-(2,2-Dimethyl-1,3-dioxan-5-yl)methoxy]-3,5-dimethylpyridin-2-yl]methyl]sulfinyl]-1*H*-benzimidazole sodium salt (racemic form)

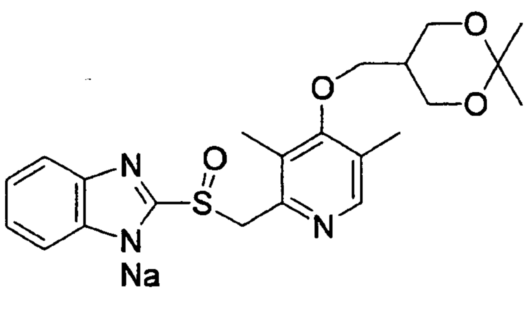

To an ethanol solution (10 mL) of 2-[[[4-(2,2-dimethyl-1,3-dioxan-5-yl)methoxy]-3,5-dimethylpyridin-2-yl]methyl] sulfinyl]-1*H*-benzimidazole (274 mg, 0.638 mmol) was added a 1 N aqueous sodium hydroxide solution (635 μL, 0.638

mmol at a concentration of 1.004 M) at room temperature followed by concentration of that mixture. Azeotropic dehydration of the residue was carried out twice with ethanol. After suspending the residue in diethyl ether and applying ultrasonic waves, the suspension was concentrated to obtain the target compound (260 mg, yield: 90.3%) as a white solid. $^1$H-NMR (400 MHz, DMSO-$d_6$) δppm: 1.33 (3H,s), 1.36 (3H,s), 2.03-2.14 (1H,m), 2.20 (3H,s), 2.21 (3H,s), 3.76-3.87 (4H, m), 4.00 (2H,dd,J=4,11 Hz), 4.39 (1H,d,J=13 Hz), 4.75 (1 H,d,J=13 Hz), 6.81-6.91 (2H,m), 7.40-7.48 (2H,m), 8.23 (1 H,s)

(Example 5) Optical Isomer Having Long Retention Time of 2-[[[4-[(2,2-dimethyl-1,3-dioxan-5-yl)methoxy]-3,5-dimethylpyridin-2-yl]methyl]sulfinyl] -1*H*-benzimidazole

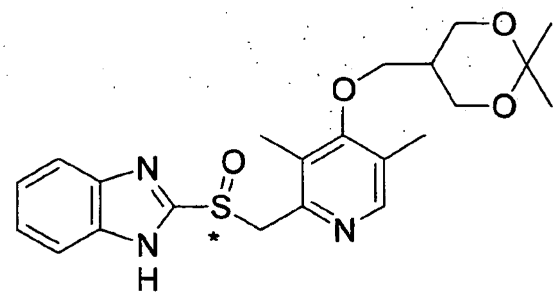

A toluene (dehydrated) (2.22 mL) - water (2.3 μL, 0.128 mmol) solution of 2-[[[4-[(2,2-dimethyl-1,3-dioxan-5-yl)methoxy]-3,5-dimethylpyridin-2-yl]methyl] thio]-1*H*-benzimidazole (444 mg, 1.07 mmol) and D-(-)-diethyl tartrate (80.6 μL, 0.471 mmol) was stirred for 10 minutes at 50°C under a nitrogen atmosphere. Titanium (IV) isopropoxide (63.2 μL, 0.214 mmol) was added thereto followed by stirring the mixture for 1 hour under the same conditions. After cooling to room temperature, N,N-diisopropyl ethylamine (59.6 μL, 0.342 mmol) was added thereto followed by cooling to 0°C. After adding cumene hydroxide (611 μL, 3.31 mmol at a content of 80%) thereto dropwise over 5 minutes at 0 to 2°C, the mixture was stirred for 3 hours 35 minutes at 0 to 7°C under a nitrogen atmosphere. After adding a saturated aqueous sodium bicarbonate solution to the reaction liquid, the mixture was extracted with ethyl acetate. The organic layer was dried over anhydrous sodium sulfate followed by filtering and concentrating. The residue was purified by silica gel column chromatography (NH silica gel: 20 g, elution solvent: dichloromethane, dichloromethane / methanol = 20 / 1). Fractions containing the target compound were collected using ethyl acetate and concentrated to obtain the target compound (388 mg, yield: 84.4%) as a colorless foam.

$^1$H-NMR (400 MHz, DMSO-$d_6$) δppm: 1.32 (3H,s), 1.36 (3H,s), 2.02-2.13 (1H,m), 2.16 (3H,s), 2.20 (3H,s), 3.74-3.85 (4H,m), 4.00 (2H,dd,J=4,12 Hz), 4.70 (1H,d,J=14 Hz), 4.79 (1H,d,J=14 Hz), 7.26-7.34 (2H,m), 7.59-7.70 (2H,m), 8.18 (1H,s)

HPLC Conditions:
Column: CHIRALPAKAD-H (Daicel) (0.46 cm ϕ x 25 cm)
Eluent: Hexane/ethanol = 1/1 (v/v)
Flow rate: 0.6 mL/min
Detection: UV 254 nm
Analysis Results:
Retention time: 17.8 minutes, enantiomeric excess: 94.4% ee

(Example 6) Sodium Salt of Optical Isomer Having Long Retention Time of 2-[[[4-[(2,2-dimethyl-1,3-dioxan-5-yl)methoxy]-3,5-dimethylpyridin-2-yl]methyl] sulfinyl]-1*H*-benzimidazole

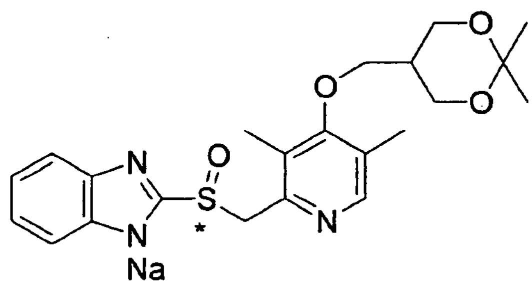

To an ethanol solution (10 mL) of an optical isomer having a long retention time of 2-[[[4-[(2,2-dimethyl-1,3-dioxan-5-yl) methoxy]-3,5-dimethylpyridin-2-yl] methyl]sulfinyl]-1*H*-benzimidazole (379 mg, 0.882 mmol) was added a 1 N aqueous sodium hydroxide solution (878 μL, 0.882 mmol at a concentration of 1.004 M) at room temperature followed by concentrating the mixture. Azeotropic dehydration of the residue was carried out with ethanol. After suspending the residue in diethyl ether and applying ultrasonic waves thereto, the suspension was concentrated to obtain the target compound (365 mg, yield: 91.7%) as a white solid.

$^1$H-NMR (400 MHz, DMSO-$d_6$) δppm: 1.33 (3H,s), 1.36 (3H,s), 2.03-2.13 (1H,m), 2.20 (3H,s), 2.21 (3H,s), 3.76-3.88 (4H,m), 4.00 (2H,dd,J=4,12 Hz), 4.38 (1H,d,J=13 Hz), 4.75 (1H,d,J=13 Hz), 6.81-6.90 (2H,m), 7.40-7.47 (2H,m), 8.23 (1 H,s)

HPLC Conditions:

Column: CHIRALPAKAD-H (Daicel) (0.46 cm ϕ x 25 cm)

Eluent: Hexane/ethanol = 1/1 (v/v)

Flow rate: 0.6 mL/min

Detection: UV 254 nm

Analysis Results:

Retention time: 17.0 minutes, enantiomeric excess: 94.9% ee

Specific rotation: αD27.4 = -76.29 (c = 0.5, EtOH)

(Example 7) Optical Isomer Having Short Retention Time of 2-[[[4-[(2,2-dimethyl-1,3-dioxan-5-yl)methoxy]-3,5-dimethylpyridin-2-yl]methyl]sulfinyl]-1*H*-benzimidazole

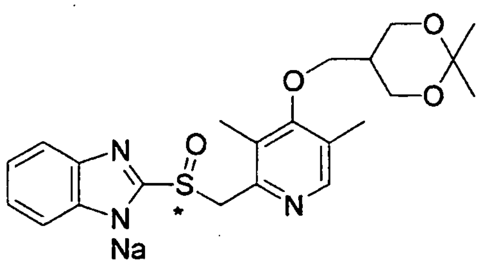

A toluene (dehydrated) (2.96 mL) - water (3.09 μL, 0.172 mmol) solution of 2-[[[4-[(2,2-dimethyl-1,3-dioxan-5-yl)methoxy]-3,5-dimethylpyridin-2-yl]methyl] thio]-1*H*-benzimidazole (591 mg, 1.43 mmol) and L-(+)-diethyl tartrate (108 μL, 0.629 mmol) was stirred for 5 minutes at 50°C in a nitrogen atmosphere. Titanium (IV) isopropoxide (84.4 μL, 0.286 mmol) was added thereto followed by stirring the mixture for 1 hour under the same conditions. After cooling to room temperature, N,N-diisopropyl ethylamine (79.7 μL, 0.458 mmol) was added thereto followed by cooling to 0°C. After adding cumene hydroxide (816 μL, 4.42 mmol at a content of 80%) dropwise thereto over 10 minutes at 0 to 1°C, the mixture was stirred for 3 hours 10 minutes under the same conditions. After adding a saturated aqueous sodium bicarbonate solution to the reaction liquid, the mixture was extracted with ethyl acetate. The organic layer was dried over anhydrous sodium sulfate followed by filtering and concentrating. The residue was purified by silica gel column chromatography (NH silica gel: 20 g, elution solvent: dichloromethane, dichloromethane/methanol = 20/1). Fractions containing the target compound were collected using ethyl acetate and concentrated to obtain the target compound (498 mg, yield: 81.1 %) as a colorless foam.

$^1$H-NMR (400 MHz, DMSO-$d_6$) δppm: 1.32 (3H,s), 1.36 (3H,s), 2.02-2.12 (1H,m), 2.16 (3H,s), 2.20 (3H,s), 3.74-3.84 (4H,m), 4.00 (2H,dd,J=4,12 Hz), 4.70 (1 H,d,J=14 Hz), 4.79 (1H,d,J=14 Hz), 7.26-7.34 (2H,m), 7.58-7.70 (2H,m), 8.18 (1 H,s)

HPLC Conditions:

Column: CHIRALPAKAD-H (Daicel) (0.46 cm ϕ x 25 cm)

Eluent: Hexane/ethanol = 1/1 (v/v)

Flow rate: 0.6 mL/min

Detection: UV 254 nm

Analysis Results:

Retention time: 14.6 minutes, enantiomeric excess: 95.4% ee

(Example 8) Sodium Salt of Optical Isomer Having Short Retention Time of 2-[[[4-[(2,2-dimethyl-1,3-dioxan-5-yl)methoxy]-3,5-dimethylpyridin-2-yl]methyl] sulfinyl]-1*H*-benzimidazole

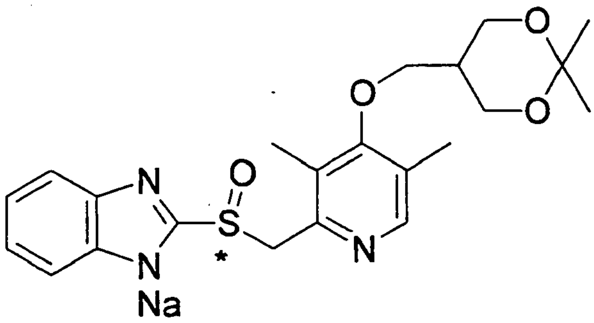

To an ethanol solution (10 mL) of the optical isomer having a short retention time of 2-[[[4-[(2,2-dimethyl-1,3-dioxan-5-yl) methoxy]-3,5-dimethylpyridin-2-yl] methyl]sulfinyl]-1*H*-benzimidazole obtained in Example 7 above (480 mg, 1.12 mmol) was added a 1 N aqueous sodium hydroxide solution (1.12 mL, 1.12 mmol at a concentration of 1.004 M) at room temperature followed by concentrating the mixture. The residue was co-boiled with ethanol. After suspending the residue in diethyl ether and applying ultrasonic waves thereto, the suspension was concentrated to obtain the target compound (447 mg, yield: 88.4%) as a white solid.

$^1$H-NMR (400 MHz, DMSO-$d_6$) δppm: 1.33 (3H,s), 1.36 (3H,s), 2.03-2.14 (1H,m), 2.21 (6H,s), 3.76-3.87 (4H,m), 4.00 (2H,dd,J=4,12 Hz), 4.39 (1H,d,J=13 Hz), 4.74 (1 H,d,J=13 Hz), 6.82-6.90 (2H,m), 7.40-7.48 (2H,m), 8.23 (1 H,s)

HPLC Conditions:

Column: CHIRALPAKAD-H (Daicel) (0.46 cm x 25 cm)

Eluent: Hexane/ethanol = 1/1 (v/v)

Flow rate: 0.6 mL/min

Detection: UV 254 nm

Analysis Results:

Retention time: 14.4 minutes, enantiomeric excess: 95.4% ee

By using 4-chloro-2-methylpyridine 1-oxide instead of the 4-chloro-2,3,5-trimethylpyridine 1-oxide of Production Example 4, or by respectively using any of the compounds represented by the following formulas instead of (2,2-dimethyl-1,3-dioxane-5-yl) methanol, as raw materials:

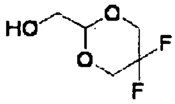
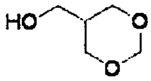
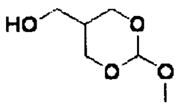
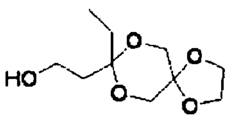
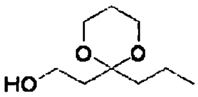
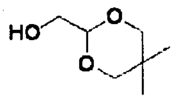
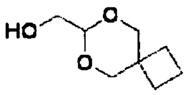
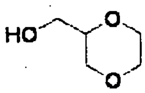
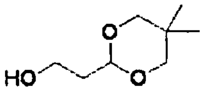
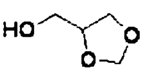
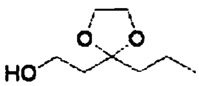
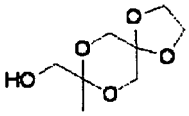
or
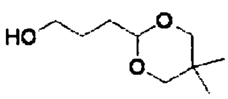

the following compounds, salts thereof (and particularly sodium salts), or optically active forms thereof, can be produced according to the same reaction conditions as described in Production Examples 5 to 7 and Examples 1 to 8.

2-[[[4-[5,5-Difluoro-1,3-dioxan-2-yl]methoxy]pyridin-2-yl]methyl]sulfinyl]-1*H*-benzimidazole

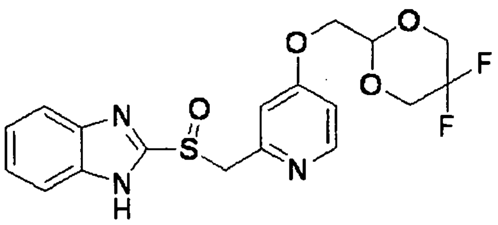

2-[[[4-[2-(2-Propyl-1,3-dioxan-2-yl)ethoxy]pyridin-2-yl]methyl]sulfinyl]-1*H*-benzimidazole

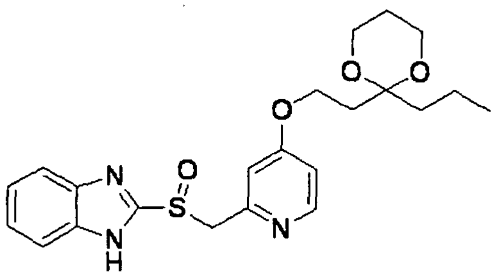

2-[[[4-(1,3-Dioxolan-4-ylmethoxy)pyridin-2-yl]methyl]sulfinyl]-1*H*-benzimidazole

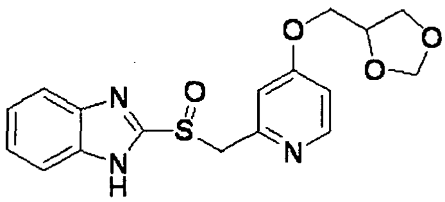

2-[[[4-(1,3-Dioxan-5-ylmethoxy)pyridin-2-yl]methyl]sulfinyl]-1*H*-benzimidazole

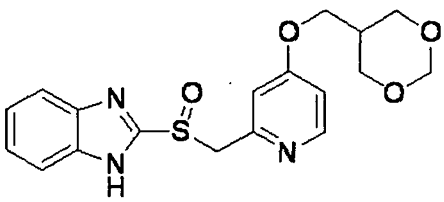

2-[[[4-[(5,5-Dimethyl-1,3-dioxan-2-yl)methoxy]pyridin-2-yl]methyl]sulfinyl]-1*H*-benzimidazole

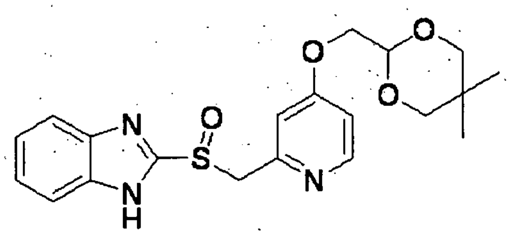

2-[[[4-[2-(2-Propyl-1,3-dioxolan-2-yl)ethoxy]pyridin-2-yl]methyl]sulfinyl]-1*H*-benzimidazole

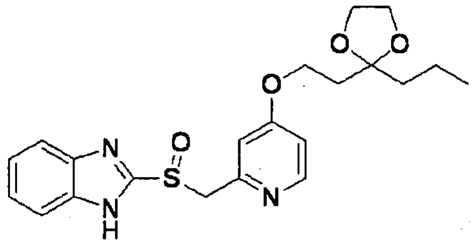

2-[[[4-[(2-Methoxy-1,3-dioxan-5-yl)methoxy]pyridin-2-yl]methyl]sulfinyl]-1*H*-benzimidazole

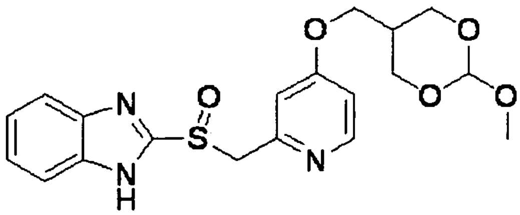

2-[[[4-(6,8-Dioxaspiro[3.5]nona-7-ylmethoxy)pyridin-2-yl]methyl]sulfinyl]-1*H*-benzimidazole

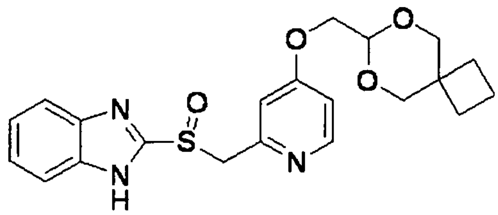

2-[[[4-[(8-Methyl-1,4,7,9-tetraoxaspiro[4.5]deca-8-yl)methoxy]pyridine-2-yl] methyl]sulfinyl]-1*H*-benzimidazole

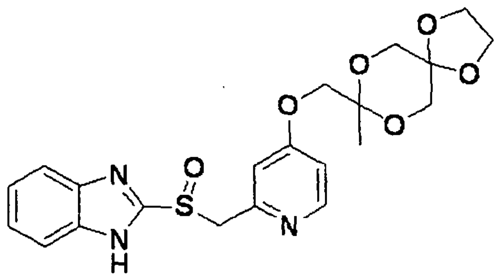

2-[[[4-[2-(8-Ethyl-1,4,7,9-tetraoxaspiro[4.5]deca-8-yl)ethoxy]pyridin-2-yl] methyl]sulfinyl]-1*H*-benzimidazole

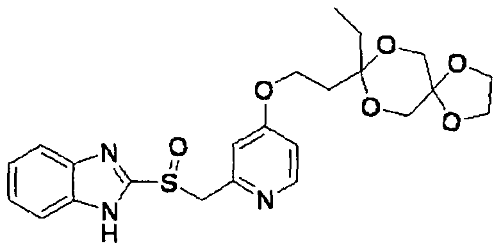

2-[[[4-(1,4-Dioxan-2-ylmethoxy)pyridin-2-yl]methyl]sulfinyl]-1*H*-benzimidazole

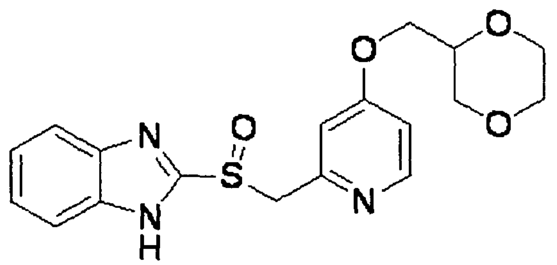

2-[[[4-[2-(5,5-Dimethyl-1,3-dioxan-2-yl)ethoxy]pyridin-2-yl]methyl]sulfinyl]-1*H*-benzimidazole

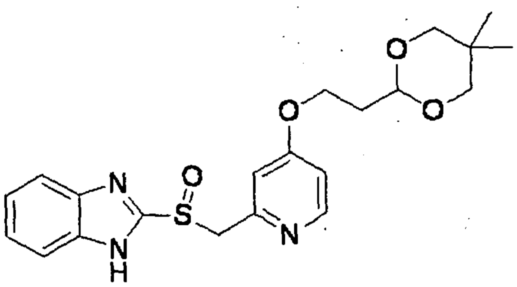

2-[[[4-[3-(5,5-Dimethyl-1,3-dioxan-2-yl)propoxy]pyridin-2-yl]methyl]sulfinyl]-1*H*-benzimidazole

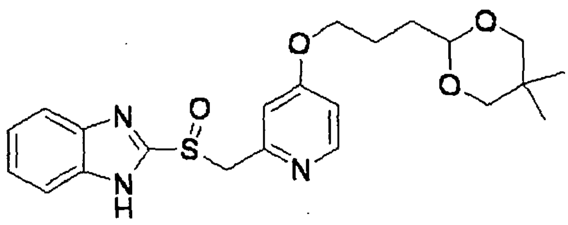

(Example 9) Sodium Salt of 2-[[[4-[3-(5,8-dioxaspiro[3.4]octa-6-yl)propoxy]-3-methylpyridin-2-yl]methyl]sulfinyl]-1*H*-benzimidazole

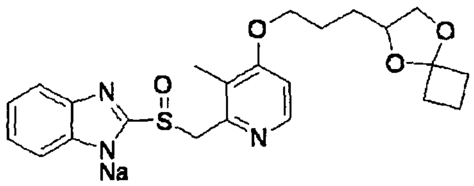

(9a): 4-[3-(2,2-Dimethyl-1,3-dioxolan-4-yl)propoxy]-2,3-dimethylpyridine 1-oxide

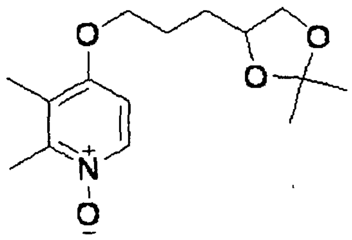

To a mixture of 3-(2,2-dimethyl-1,3-dioxolan-4-yl) propan-1-ol (5 g, 31.2 mmol) (synthesized with reference to the method described in Tetrahedron, 59(2003), 5861-5868) and dimethylsulfoxide (60 mL) was added sodium hydride in oil (1.5 g, 37.4 mmol for a content of 60%) gradually added at room temperature, which was stirred for 30 minutes at room temperature. To the reaction mixture was added 4-chloro-2,3-dimethylpyridine 1-oxide (5.41 g, 34.3 mmol) at room temperature, which was stirred for 2 hours at 60°C. After concentrating the reaction mixture, the resulting residue was purified by silica gel column chromatography (NH silica gel, elution solvent: ethyl/methanol = 1/0 to 4/1 gradient) to obtain the target compound (5.56 g, yield: 63.3%) as an orange oily substance.

$^1$H-NMR (400 MHz, DMSO-$d_6$) δppm: 1.24 (3H,s), 1.29 (3H,s), 1.55-1.86 (4H,m), 2.11 (3H,s), 2.33 (3H,s), 3.40-3.48 (1H,m), 3.94-4.12 (4H,m), 6.90 (1H,d,J=7 Hz), 8.06 (1 H,d,J=7 Hz)

(9b): [4-[3-(2,2-Dimethyl-1,3-dioxolan-4-yl)propoxy]-3-methylpyridin-2-yl] methanol

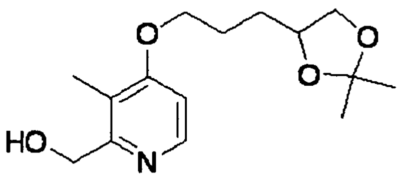

A mixture of 4-[3-(2,2-dimethyl-1,3-dioxolan-4-yl)propoxy]-2,3- dimethylpyridine 1-oxide obtained in (9a) above (5.56 g, 19.8 mmol) and acetic anhydride (45 mL) was stirred for 30 minutes at 85°C. After concentrating the reaction mixture, methanol (45 mL) was added to the resulting residue followed by the addition of a 5 N aqueous sodium hydroxide solution (30 mL) and stirring for 30 minutes at room temperature. A saturated aqueous ammonium chloride solution (30

mL) was added to the reaction mixture to adjust the pH to about 10. The mixture was concentrated followed by the addition of ethyl acetate. This mixture was then sequentially washed with 2 N aqueous sodium hydroxide solution, water and saturated brine followed by drying over anhydrous sodium sulfate and concentrating to obtain the target compound (4.10 g, yield: 73.6%) as an orange oily substance.
$^1$H-NMR (400 MHz, DMSO-$d_6$) δppm: 1.24 (3H,s), 1.29 (3H,s), 1.57-1.88 (4H,m), 2.09 (3H,s), 3.45 (1 H,t,J=7 Hz), 3.95-4.14 (4H,m), 4.50 (2H,d,J=5 Hz), 4.96 (1 H,t,J=5 Hz), 6.91 (1 H,d,J=6 Hz), 8.20 (1 H,d,J=6 Hz)

(9c): 2-[[[4-[3-(2,2-Dimethyl-1,3-dioxolan-4-yl)propoxy]-3-methylpyridin-2-yl] methyl]thio]-1*H*-benzimidazole

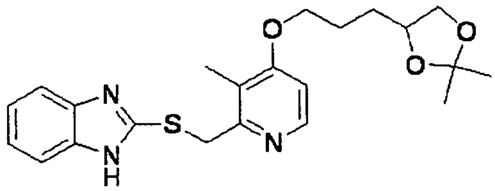

To a mixture of the [4-[3-(2,2-dimethyl-1,3-dioxolan-4-yl)propoxy]-3-methylpyridin-2-yl] methanol obtained in (9b) above (2.08 g, 7.39 mmol) and tetrahydrofuran (40 mL) was added triethylamine (2.1 mL, 15.1 mmol) at room temperature, followed by the addition of methanesulfonyl chloride (860 μL, 11.1 mmol) while cooling on an ice/salt bath. The reaction mixture was stirred for 30 minutes while cooling on an ice/salt bath. 2-Mercaptobenzimidazole (1.11 g 7.39 mmol) was added to the reaction mixture at room temperature followed by stirring for 16 hours. After concentrating the reaction mixture, NH silica gel was added to the resulting residue followed by concentrating and drying to a solid and purifying by silica gel column chromatography (silica gel, elution solvent: heptane/ethyl acetate = 1/0 → 1/1-1/2 gradient) to obtain the target compound (2.22 g, yield: 72.6%) as a light yellow foam.
$^1$H-NMR (400 MHz, DMSO-$d_6$) δppm: 1.24 (3H,s), 1.29 (3H,s), 1.57-1.89 (4H,m), 2.19 (3H,s), 3.45 (1 H,t,J=7 Hz), 3.96-4.13 (4H,m), 4.67 (2H,s), 6.92 (1 H,d,J=6 Hz), 7.06-7.14 (2H,m), 7.43 (2H,brs), 8.21 (1 H,d,J=6 Hz), 12.60 (1 H,brs)

(9d): 5-[[2-[(1H-benzimidazol-2-ylthio)methyl]-3-methylpyridin-4-yl]oxy] pentane-1,2-diol

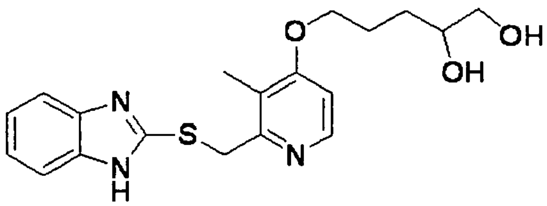

To a mixture of 2-[[[4-[3-(2,2-dimethyl-1,3-dioxolan-4-yl)propoxy]-3-methylpyridin-2-yl]methyl]thio]-1*H*-benzimidazole obtained in (9c) above (1.54 g, 3.73 mmol) and methanol (10 mL) was added 2 N hydrochloric acid (10 mL), which was stirred for 1.5 hours at room temperature. A 2 N aqueous sodium hydroxide solution was added to the reaction mixture so that the pH of the reaction mixture became nearly neutral followed by stirring for 5 hours at room temperature. The white solid that formed was filtered and dried for 17 hours at 50°C to obtain the target compound (1.224 g, yield: 87.9%) as a white solid.
$^1$H-NMR (400 MHz, DMSO-$d_6$) δppm: 1.28-1.40 (1H,m), 1.55-1.93 (3H,m), 2.19 (3H,s), 3.17-3.33 (2H,m), 3.38-3.48 (1H,m), 4.05 (2H,t,J=6 Hz), 4.42-4.52 (2H,br), 4.67 (2H,s), 6.93 (1 H,d,J=6 Hz), 7.06-7.14 (2H,m), 7.37-7.48 (2H,br), 8.21 (1 H,d,J=6 Hz), 12.60 (1H,brs)

(9e): 2-[[[4-[3-(5,8-Dioxaspiro[3.4]octa-6-yl)propoxy]-3-methylpyridin-2-yl] methyl]thio]-1*H*-benzimidazole

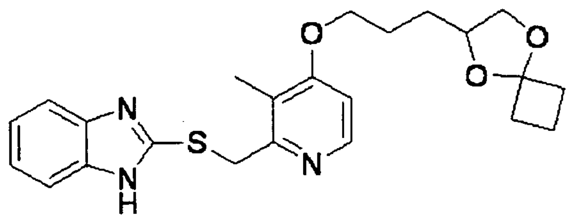

To a mixture of iron (III) chloride (109 mg, 0.67 mmol) and cyclobutanone (1 mL, 13.4 mmol) was added 5-[[2-[(1*H*-benzimidazol-2-ylthio)methyl]-3-methylpyridin-4-yl]oxy]pentane-1,2-diol obtained in (9d) above (250 mg, 0.67 mmol), which was stirred for 15 minutes at room temperature. Iron (III) chloride (54 mg, 0.34 mmol) was further added to this reaction mixture followed by stirring for 2 hours at room temperature. Iron (III) chloride (54 mg, 0.34 mmol) was again added to this reaction mixture followed by stirring for 1 hour at room temperature and then for 1 hour at 50°C. NH silica gel was added to the reaction mixture followed by concentrating and drying to a solid and purifying by silica gel column chromatography (NH silica gel, elution solvent: heptane/ethyl acetate = 1/0 → 1/1-0/1 gradient) to obtain the target compound (214 mg, yield: 75.1 %) as a colorless oily substance.

$^1$H-NMR (400 MHz, DMSO-$d_6$) δppm: 1.50-1.90 (6H,m), 2.09-2.26 (4H,m), 2.19 (3H,s), 3.39-3.46 (1H,m), 3.90-4.14 (4H,m), 4.67 (2H,s), 6.93 (1H,d,J=6 Hz), 7.06-7.14 (2H,m), 7.43 (2H,brs), 8.22 (1 H,d,J=6 Hz), 12.59 (1 H,brs)

(9f): 2-[[[4-[3-(5,8-Dioxaspiro[3.4]octa-6-yl)propoxy]-3-methylpyridin-2-yl] methyl]sulfinyl]-1*H*-benzimidazole

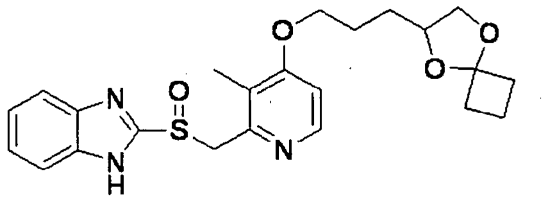

to the 2-[[[4-[3-(5,8-dioxaspiro[3.4]octa-6-yl)propoxy]-3-methylpyridin-2-yl]methyl]thio]-1*H*-benzimidazole obtained in (9e) above (212 mg, 0.498 mmol) was added a mixed solvent (10 mL) of toluene and methanol (9/1), which cooled to -65°C on a methanol-water-dry ice bath (Mixture A1).

To 3-chlorobenzoic acid (119 mg, 0.448 mmol for a content of 65%) (Mixture B1) was added a mixed solvent (2 mL) of toluene and methanol (9/1).

Into the above Mixture A1 was added the above Mixture B1 dropwise over 10 minutes at -65 to -60°C followed by stirring for 1 hour 30 minutes at -70 to -60°C. A saturated aqueous sodium bicarbonate solution (12 mL) was added to the reaction mixture at -70 to -60°C. After adding chloroform (50 mL) to the reaction mixture and stirring well, the organic layer was obtained by separation followed by the further addition of chloroform (50 mL) to the aqueous layer, stirring well and obtaining the organic layer by separation. The combined organic layers were dried over anhydrous sodium sulfate and concentrated. The resulting residue was purified by silica gel column chromatography (NH silica gel, elution solvent: ethyl acetate/methanol = 1/0-4/1 gradient) to obtain the target compound (180 mg, content: 94.8%, yield: 77.6%) as a black oily substance.

$^1$H-NMR (400 MHz, DMSO-$d_6$) δppm: 1.50-1.96 (6H,m), 2.07-2.26 (4H,m), 2.11 (3H,s), 3.42 (1H,t,J=7 Hz), 3.87-4.14 (4H,m), 4.68 (1H,d,J=14 Hz), 4.78 (1H,d,J=14 Hz), 6.93 (1 H,d,J=6 Hz), 7.22-7.34 (2H,m), 7.55-7.70 (2H,br), 8.20 (1 H, d,J=6 Hz)

(9g): 2-[[[4-[3-(5,8-Dioxaspiro[3.4]octa-6-yl)propoxy]-3-methylpyridin-2-yl] methyl]sulfinyl]-1*H*-benzimidazole sodium salt

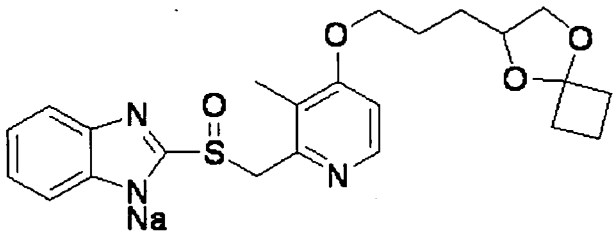

To a mixture of 2-[[[4-[3-(5,8-dioxaspiro[3.4]octa-6-yl)propoxy]-3-methylpyridin-2-yl] methyl]sulfinyl]-1 H-benzimidazole obtained in (9f) above (179 mg, content: 94.8%, 0.384 mmol) and ethanol (5 mL) was added a 1 N aqueous sodium hydroxide solution (384 μL, 0.384 mmol), which was stirred for 15 minutes at room temperature. The reaction mixture was concentrated and methanol was added to the resulting residue followed by concentration of the mixture. The addition of methanol to the residue and the subsequent concentration procedure were further carried out twice in the same manner. Diethyl ether was then added to the resulting residue, and the supernatant of the suspension that formed was removed and concentrated followed by further evaporating the solvent with a vacuum pump to obtain the target compound (170 mg, yield: 95.5%) as a light yellow solid. $^1$H-NMR (400 MHz, DMSO-$d_6$) δppm: 1.50-1.90 (6H,m), 2.09-2.29 (4H,m), 2.16 (3H,s), 3.42 (1 H,t,J=7 Hz), 3.88-4.14 (4H,m), 4.38 (0.5H,d,J=13 Hz), 4.39 (0.5H,d,J=13 Hz), 4.76 (0.5H,d,J=13 Hz), 4.77 (0.5H,d,J=13 Hz), 6.77-6.96 (3H,m), 7.35-7.50 (2H,m), 8.26 (1H,d,J=5 Hz)

(Example 10) Sodium Salt of 2-[[[4-[2-(5,8-dioxaspiro[3.4]octa-6-yl)ethoxy]-3-methylpyridin-2-yl]methyl]sulfinyl]-1*H*-benzimidazole

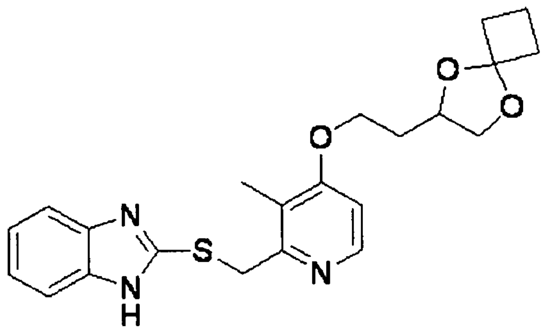

(10a): 2-(2,2-Dimethyl-1,3-dioxolan-4-yl) ethanol

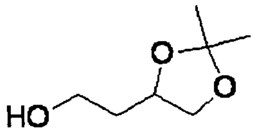

A mixture of 1,2,4-butanetriol (15 g, 141 mmol), acetone (100 mL, 1362 mmol) and p-toluenesulfonic acid monohydrate (700 mg, 3.68 mmol) was stirred for 16 hours at room temperature. Triethylamine (2 mL, 14.3 mmol) was added to the reaction mixture and concentrated. The resulting residue was purified by silica gel column chromatography (silica gel, elution solvent: heptane/ethyl acetate = 1/0 → 1/1-1/3 gradient) to obtain the target compound (16.55 g, yield: 80.3%) as a colorless oily substance.

$^1$H-NMR (400 MHz, $CDCl_3$) δppm: 1.37 (3H,s), 1.43 (3H,s), 1.79-1.86 (2H,m), 2.18-2.24 (1 H,br), 3.60 (1 H,dd,J=7, 8 Hz), 3.76-3.86 (2H,m), 4.10 (1 H,dd,J= 6, 8 Hz), 4.23-4.32 (1 H,m)

(10b): 4-[2-(2,2-Dimethyl-1,3-dioxolan-4-yl)ethoxy]-2,3-dimethylpyridine 1-oxide

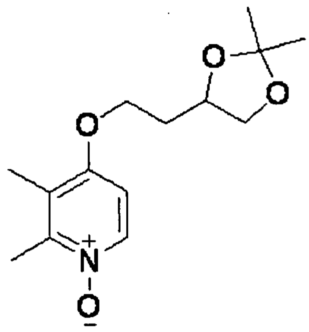

To a mixture of 2-(2,2-dimethyl-1,3-dioxolan-4-yl) ethanol (16.52 g) obtained in (10a) above, 2-(2,2-dimethyl-1,3-dioxolan-4-yl) (5.18 g) (total: 21.7 g, 148 mmol) and dimethylsulfoxide (220 mL) was added sodium hydride in oil (7.1 g, 178 mmol for a content of 60%) gradually at room temperature, which was stirred for 30 minutes at room temperature. 4-Chloro-2,3-dimethylpyridine 1-oxide (23.35 g, 148 mmol) was added to the reaction mixture followed by stirring for 2 hours at 60°C. The reaction mixture was concentrated after which the resulting residue was purified by silica gel column chromatography (NH silica gel, elution solvent: heptane/ethyl acetate = 1/1 → ethyl acetate/methanol = 1/0-4/1 gradient) to obtain the target compound (25.25 g, yield: 63.8%) as an orange oily substance.

$^1$H-NMR (400 MHz, DMSO-$d_6$) δppm: 1.25 (3H,s), 1.31 (3H,s), 1.89-2.01 (2H,m), 2.11 (3H,s), 2.33 (3H,s), 3.56 (1 H, dd,J=7, 8 Hz), 3.94-4.24 (4H,m), 6.92 (1 H,d,J=7 Hz), 8.05 (1 H,d,J=7 Hz)

(10c): [4-[2-(2,2-Dimethyl-1,3-dioxolan-4-yl)ethoxy]-3-methylpyridine-2-yl] methanol

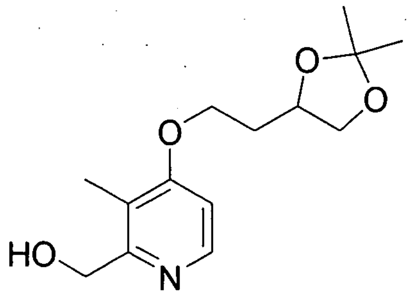

A mixture of 4-[2-(2,2-dimethyl-1,3-dioxolan-4-yl)ethoxy]-2,3- dimethylpyridine 1-oxide obtained in (10b) above (25.3 g, 94.6 mmol) and acetic anhydride (210 mL) was stirred for 1 hour at 85°C. After concentrating the reaction mixture, methanol (210 mL) was added to the resulting residue followed by the addition of 5 N aqueous sodium hydroxide solution (140 mL) and stirring for 30 minutes at room temperature. Saturated aqueous ammonium chloride solution (140 mL) was added to the reaction mixture to adjust to a pH of about 10. The mixture was concentrated followed by the addition of ethyl acetate. The organic layer was washed with 2 N aqueous sodium hydroxide solution, water and saturated brine followed by drying over anhydrous sodium sulfate and concentrating to obtain the target compound (17.89 g, yield: 70.7%) as a brown oily substance.

$^1$H-NMR (400 MHz, DMSO-$d_6$) δppm: 1.25 (3H,s), 1.31 (3H,s), 1.91-2.02 (2H,m), 2.09 (3H,s), 3.54-3.60 (1H,m), 3.98-4.26 (4H,m), 4.51 (2H,d,J=6 Hz), 4.96 (1H,t,J=6 Hz), 6.93 (1 H,d,J=6 Hz), 8.21 (1 H,d,J=6 Hz)

(10d): 2-[[[4-[2-(2,2-Dimethyl-1, 3-dioxoian-4-yl)ethoxy]-3-methylpyridin-2-yl] methyl]thio]-1*H*-benzimidazole

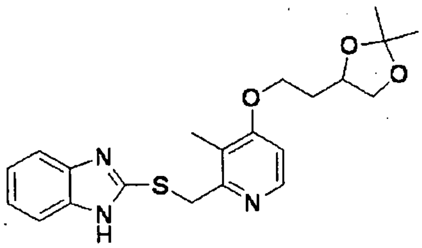

To a mixture of [4-[2-(2,2-dimethyl-1,3-dioxolan-4-yl)ethoxy]-3-methylpyridin-2-yl]methanol obtained in (10c) above (12.9 g, 48.2 mmol) and tetrahydrofuran (220 mL) was added triethylamine (13.5 mL, 96.9 mmol) at room temperature. Methanesulfonyl chloride (5.5 mL, 71.1 mmol) was added to the reaction mixture while cooling on an ice/salt bath. The reaction mixture was then stirred for 30 minutes while cooling on an ice/salt bath. 2-Mercaptobenzimidazole (7.24 g, 48.2 mmol) was added to the reaction mixture at room temperature followed by stirring for 16.5 hours. The reaction mixture was concentrated followed by the addition of NH silica gel to the resulting residue, concentrating, drying to a solid and purifying by silica gel column chromatography (silica gel, elution solvent: heptane/ethyl acetate = 1/0 → 1/1-0/1 gradient) to obtain the target compound (16.5 g, yield: 85.7%) as a light yellow foam.
$^1$H-NMR (400 MHz, DMSO-$d_6$) δppm: 1.25 (3H,s), 1.31 (3H,s), 1.90-2.04 (2H,m), 2.20 (3H,s), 3.57 (1 H,dd,J=7, 8 Hz), 3.98-4.26 (4H,m), 4.68 (2H,s), 6.95 (1 H,d,J=6 Hz), 7.07-7.14 (2H,m), 7.38-7.49 (2H,br), 8.22 (1 H,d,J=6 Hz), 12.60 (1 H,brs)

(10e): 4-[[2-[(1*H*-benzimidazol-2-ylthio)methyl]-3-methylpyridin-4-yl]oxy] butane-1,2-diol

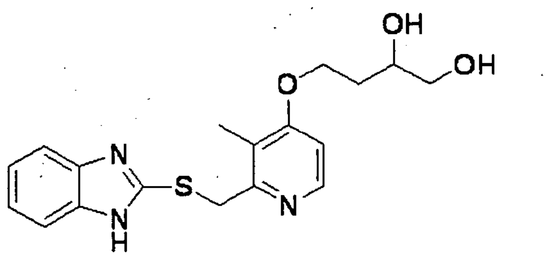

To a mixture of 2-[[[4-[2-(2,2- dimethyl-1,3-dioxolan-4-yl)ethoxy]-3-methylpyridin-2-yl]methyl]thio]-1*H*-benzimidazole (16.5 g, 41.3 mmol) obtained in (10d) above and methanol (100 mL) was added 2 N hydrochloric acid (100 mL), which was stirred for 1.5 hours at room temperature. 2 N aqueous sodium hydroxide solution was added to the reaction mixture so that the pH of the reaction mixture was nearly neutral followed by stirring for 4 hours at room temperature. A white solid that had formed in the reaction mixture was filtered out. Methanol was added to the resulting solid (and after stirring to mix well and sonicating) followed by filtering out the solid and air-drying for 10 hours at 50°C to obtain the target compound (11.20 g) as a white solid (Lot A).
The mother liquor was then concentrated. The resulting residue was suspended in methanol and filtered followed by air-drying for 24 hours at 50°C to obtain the target compound (1.67 g) as a light yellow solid (Lot B). (Total of Lot A and Lot B: 12.87 g, yield: 86.7%)
A mixture of the target compounds in the form of Lot A (8.76 g) and Lot B (1.17 g) along with methanol (160 mL) was stirred while heating and refluxing. After stirring the reaction mixture for 2 hours 30 minutes at room temperature, the solid that precipitated in the mixture was filtered. Moreover, methanol (125 mL) was added to the resulting solid followed by stirring while heating and refluxing. After stirring the reaction mixture for 14 hours at room temperature, the precipitated solid was filtered to obtain the target compound (5.53 g, recovery rate: 55.7%) (Lot C).
$^1$H-NMR (400 MHz, DMSO-$d_6$) δppm: 1.59-1.70 (1 H,m), 1.90-2.00 (1 H,m), 2.19 (3H,s), 3.25-3.40 (2H,m), 3.59-3.69 (1H,m), 4.10-4.18 (2H,m), 4.52-4.58 (1 H,br), 4.60-4.66 (1 H,br), 4.67 (2H,s), 6.94 (1 H,d,J=6 Hz), 7.06-7.14 (2H,m), 7.43 (2H,brs), 8.22 (1 H,d,J=6 Hz), 12.60 (1 H,brs)

(10f): 2-[[[4-[2-(5,8-Dioxaspiro[3.4]octa-6-yl)ethoxy]-3-methylpyridin-2-yl] methyl]thio]-1*H*-benzimidazole

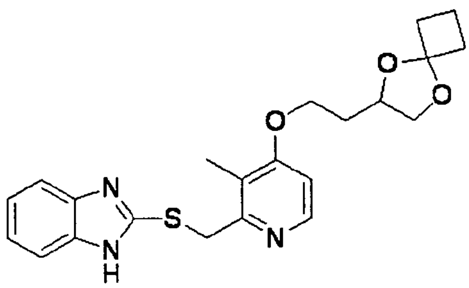

To a mixture of iron (III) chloride (360 mg, 2.22 mol) and cyclobutanone (2 mL, 26.8 mmol) was added

4-[[2-[(1*H*-benzimidazol-2-ylthio)methyl]-3-methylpyridin-4-yl]oxy]butane-1,2-diol obtained in (10e) above (Lot A, 400 mg, 1.11 mmol), which was stirred for 30 minutes at 50°C. NH silica gel was added to the reaction mixture followed by concentrating, drying to a solid and purifying by silica gel column chromatography (NH silica gel, elution solvent: heptane/ethyl acetate = 1/0 → 1/1-0/1 gradient) to obtain the target compound (390 mg, yield: 67.6%) as a colorless oily substance. $^1$H-NMR (400 MHz, DMSO-$d_6$) δppm: 1.57 (2H,quint,J=8 Hz), 1.90-2.02 (2H,m), 2.11-2.30 (4H,m), 2.20 (3H,s), 3.54 (1 H,dd,J=6, 8 Hz), 3.98 (1H,dd,J=6, 8 Hz), 4.07-4.22 (3H,m), 4.68 (2H,s), 6.95 (1 H,d,J=6 Hz), 7.06-7.14 (2H,m), 7.38-7.48 (2H,m), 8.22 (1 H,d,J=6 Hz)

(10g): 2-[[[4-[2-(5,8)-Dioxaspiro[3.4]octa-6-yl]ethoxy]-3-methylpyridine-2-yl] methyl]sulfinyl]-1*H*-benzimidazole

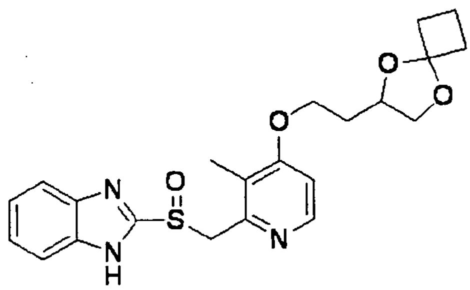

A mixture of the 2-[[[4-[2-(5,8-dioxaspiro[3.4]octa-6-yl)ethoxy]-3-methylpyridin-2-yl]methyl]thio]-1H-benzimidazole obtained in (10f) above (305 mg, 0.741 mmol) and a mixed solvent (10 mL) of toluene and methanol (9/1) was cooled to -65°C with a methanol/water/dry ice bath (Mixture A2).

To 3-perchlorobenzoic acid (177 mg, 0.667 mol for a content of 65%) was added a mixed solvent (2 mL) of toluene and methanol (9/1) (Mixture B2).

The above Mixture B2 was added to the Mixture A2 dropwise over 10 minutes at -65 to -60°C followed by stirring for 1 hour at -65 to -60°C. Saturated aqueous sodium bicarbonate solution (12 mL) was added to the reaction mixture at -65 to -60°C. After adding chloroform (50 mL) to the reaction mixture and stirring well, the organic layer was removed followed by further addition of chloroform (50 mL) to the aqueous layer, stirring well and removing the organic layer. The combined organic layers were concentrated after drying over anhydrous sodium sulfate. The resulting residue was purified by silica gel column chromatography (NH silica gel, elution solvent: ethyl acetate/methanol = 1/0-4/1 gradient) to obtain the target compound (280 mg, content: 97.3%, yield: 86%) as a light violet foam.

$^1$H-NMR (400 MHz, DMSO-$d_6$) δppm: 1.57 (2H,quint,J=8 Hz), 1.91-2.00 (2H,m), 2.12 (3H,s), 2.13-2.30 (4H,m), 3.53 (1 H,dd,J= 7, 8 Hz), 3.93-4.22 (4H,m), 4.69 (1 H,d,J=14 Hz), 4.78 (0.5H,d,J=14 Hz), 4.78 (0.5H,d,J=14 Hz), 6.95 (1H,d, J=6 Hz), 7.24-7.32 (2H,m), 7.57-7.69 (2H,br), 8.21 (1 H,d,J=6 Hz), 13.55 (1 H,brs)

(10h): Sodium Salt of 2-[[[4-[2-(5,8-dioxaspiro[3.4]octa-6-yl)ethoxy]-3-methylpyridin-2-yl]methyl]sulfinyl]-1*H*-benzimidazole

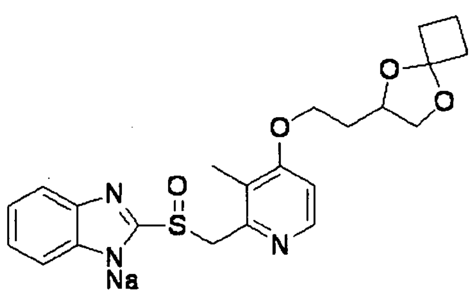

To a mixture of 2-[[[4-[2-(5,8-dioxaspiro[3.4]octa-6-yl)ethoxy]-3-methylpyridine-2-yl]methyl]sulfinyl]-1*H*-benzimidazole obtained in (10g) above (278 mg, 0.632 mmol for a content of 97.3%) and ethanol (5 mL) was added a 1 N aqueous sodium hydroxide solution (631 μL, 0.632 mmol), which was stirred for 15 minutes at room temperature followed by concentrating the reaction mixture. Methanol was added to the resulting residue followed by concentration of the mixture. Methanol was added to the residue followed by carrying out the concentration procedure two more times in the same manner. Diethyl ether was then added to the resulting residue and the supernatant of the suspension that formed

was removed and concentrated followed by further distilling off the solvent with a vacuum pump to obtain the target compound (282 mg, yield: 99.3%) as a light yellow solid.
$^{1}$H-NMR (400 MHz, DMSO-d$_6$) δppm: 1.57 (2H,quint,J=8 Hz), 1.88-2.04 (2H,m), 2.10-2.32 (4H,m), 2.16 (3H,s), 3.54 (1 H,t,J=7 Hz), 3.90-4.23 (4H,m), 4.38 (0.5H,d,J=13 Hz), 4.39 (0.5H,d,J=13 Hz), 4.71-4.82 (1H,m), 6.76-6.97 (3H,m), 7.35-7.50 (2H,m), 8.27 (1 H,d,J=6 Hz)

(Example 11) Sodium Salt of 2-[[[4-[2-(1,4-dioxaspiro[4.4]nona-2-yl)ethoxy]-3-methylpyridin-2-yl]methyl]sulfinyl]-1*H*-benzimidazole

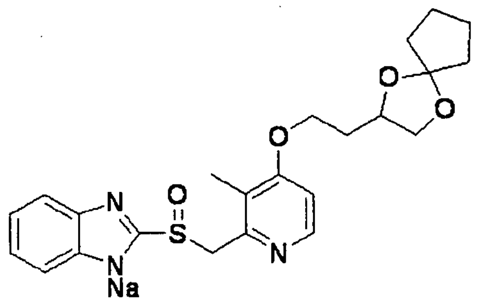

The target compound (206 mg, total yield: 40.6%) was obtained as a light yellow solid by carrying out the procedure under the same conditions as (10f) to (10h) using an equimolar amount of cyclopentanone instead of the cyclobutanone in (10f) above.
$^{1}$H-NMR (400 MHz, DMSO-d$_6$) δppm: 1.50-1.77 (8H,m), 1.88-2.03 (2H,m), 2.17 (3H,s), 3.55 (1 H,t,J=7 Hz), 3.99 (1 H, t,J=7 Hz), 4.04-4.22 (3H,m), 4.37 (0.5H,d,J=13 Hz), 4.38 (0.5H,d,J=13 Hz), 4.77 (0.5H,d,J=13 Hz), 4.78 (0.5H,d,J=13 Hz), 6.78-6.90 (2H,m), 6.91 (1 H,d,J=5 Hz), 7.36-7.48 (2H,m), 8.26 (1 H,d,J=5 Hz)

(Example 12) Sodium Salt of 2-[[[4-[2-(1,4-dioxaspiro[4.5]deca-2-yl)ethoxy]-3-methylpyridin-2-yl]methyl]sulfinyl]-1*H*-benzimidazole

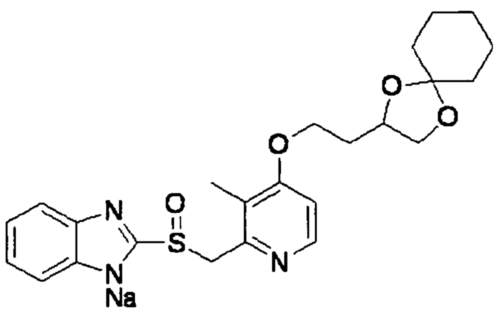

The target compound (262 mg, total yield: 51.3%) was obtained as a light yellow solid by carrying out the procedure under the same conditions as (10f) to (10h) using an equimolar amount of cyclohexanone instead of the cyclobutanone in (10f) above.
$^{1}$H-NMR (400 MHz, DMSO-d$_6$) δppm: 1.20-1.59 (10H,m), 1.92-2.03 (2H,m), 2.17 (3H,s), 3.57 (1H,t,J=7 Hz), 3.94-4.26 (4H,m), 4.37 (0.5H,d,J=13 Hz), 4.38 (0.5H,d,J=13 Hz), 4.77 (0.5H,d,J=13 Hz), 4.78 (0.5H,d,J=13 Hz), 6.78-6.89 (2H, m), 6.92 (1 H,d,J=6 Hz), 7.36-7.48 (2H,m), 8.26 (1 H,d,J=6 Hz)

(Example 13) Sodium Salt of 2-[[[3-methyl-4-[2-(1,4,8-trioxaspiro[4.5]deca-2-yl)ethoxy]pyridin-2-yl]methyl]sulfinyl]-1*H*-benzimidazole

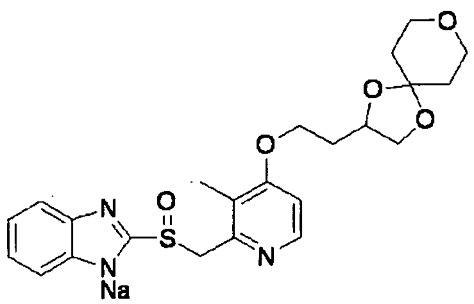

The target compound (211 mg, total yield: 32.1 %) was obtained as a light yellow solid by carrying out the procedure under the same conditions as (10f) to (10h) using an equimolar amount of tetrahydro-4*H*-pyran-4-one instead of the cyclobutanone in (10f) above.
$^1$H-NMR (400 MHz, DMSO-$d_6$) δppm: 1.55-1.72 (4H,m), 1.94-2.06 (2H,m), 2.17 (3H,s), 3.50-3.70 (5H,m), 4.04-4.19 (3H,m), 4.21-4.32 (1 H,m), 4.36 (0.5H,d,J=13 Hz), 4.38 (0.5H,d,J=13 Hz), 4.79 (0.5H,d,J=13 Hz), 4.79 (0.5H,d,J=13 Hz), 6.78-6.88 (2H,m), 6.92 (1 H,d,J=6 Hz), 7.37-7.47 (2H,m), 8.26 (1 H,d,J=6 Hz)

(Example 14) Sodium Salt of 2-[[[4-[2-(2,2-diethyl-1,3-dioxolan-4-yl)ethoxy]-3-methylpyridin-2-yl]methyl]sulfinyl]-1*H*-benzimidazole

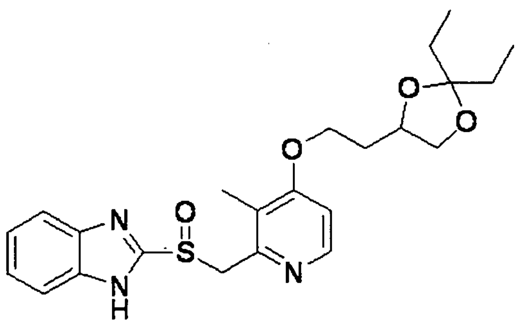

The target compound (155 mg, total yield: 24.4%) was obtained as a light yellow solid by carrying out the procedure under the same conditions as (10f) to (10h) using an equimolar amount of 3-pentanone instead of the cyclobutanone in (10f) above.
$^1$H-NMR (400 MHz, DMSO-$d_6$) δppm: 0.80 (3H,t,J=7 Hz), 0.82 (3H,t,J=7 Hz), 1.52 (2H,q,J=7 Hz), 1.55 (2H,q,J=7 Hz), 1.91-2.06 (2H,m), 2.16 (3H,s), 3.52 (1H,t,J=8 Hz), 3.99-4.27 (4H,m), 4.37 (0.5H,d,J=13 Hz), 4.38 (0.5H,d,J=13 Hz), 4.76 (0.5H,d,J=13 Hz), 4.77 (0.5H,d,J=13 Hz), 6.79-6.90 (2H,m), 6.92 (1 H,d,J=6 Hz), 7.36-7.49 (2H,m), 8.26 (1 H,d,J=6 Hz)

(Example 15) Sodium Salt of 2-[[[4-[3-hydroxy-2-(hydroxymethyl)propoxy]-3,5-dimethylpyridin-2-yl]methyl] sulfinyl]-1*H*-benzimidazole

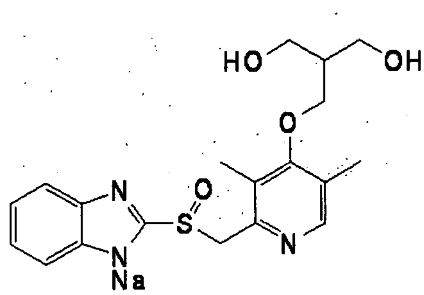

(15a): 2-[[[2-[(1*H*-benzimidazol-2-ylthio)methyl]-3,5-dimethylpyridin-4-yl]-oxy] methyl]propane-1,3-diol

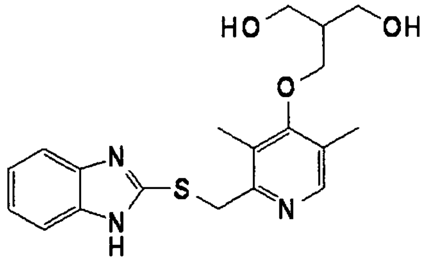

To a mixture of 2-[[[4-(5,9-dioxaspiro [3.5]nona-7-ylmethoxy)-3,5-dimethylpyridin-2-yl]methyl]thio]-1*H*-benzimidazole (80 mg, 0.188 mmol) and methanol (0.5 mL) was added 2 N hydrochloric acid (0.5 mL), which was stirred for 1.5 hours at room temperature. A 2 N aqueous sodium hydroxide solution (0.5 mL) was added to the reaction mixture followed by stirring for about 30 minutes. The precipitate that precipitated in the mixture was filtered out and washed with water. After adding methanol to the precipitate, the solvent was evaporated under a reduced pressure followed by further evaporating the solvent from the resulting residue with a vacuum pump to obtain the target compound (50 mg, yield: 71.2%) as a white solid.
$^1$H-NMR (400 MHz, DMSO-$d_6$) δppm: 1.93-2.03 (1H,m), 2.18 (3H,s), 2.27 (3H,s), 3.50-3.61 (4H,m), 3.82 (2H,d,J=6 Hz), 4.53 (2H,brs), 4.66 (2H,s), 7.07-7.14 (2H,m), 7.38-7.48 (2H,m), 8.14 (1 H,s)
(15b): 2-[[[2-[(1*H*-benzimidazol-2-ylsulfinyl)methyl]-3,5-dimethylpyridin-4-yl]-oxy]methyl]propane-1,3-diol
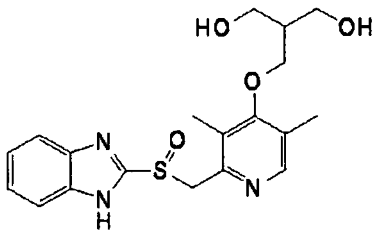

To a mixture of 2-[[[2-[(1H-benzimidazol-2-ylthio) methyl]-3,5-dimethylpyridin-4-yl]-oxy]methyl]propane-1,3-diol obtained according to the method of (9a) above (2 g, 5.36 mmol), methanol (60 mL) and toluene (100 mL) was added a methanol (2 mL) solution of 3-chloroperbenzoic acid (1.28 g, 4.82 mmol for a content of 65%) at -60°C (internal temperature) dropwise, which was stirred for 2.5 hours at -60°C (internal temperature). Saturated aqueous sodium bicarbonate solution was added to the reaction mixture followed by stirring well and removing the organic layer. Ethyl acetate was similarly added to the aqueous layer followed by carrying out the procedure of stirring well and removing the organic layer two more times. The combined organic layers were washed with saturated aqueous sodium carbonate solution and saturated brine followed by drying over sodium sulfate and concentrating. The residue was then purified by silica gel column chromatography (NH silica gel, elution solvent: dichloromethane, 10% methanol in dichloromethane). Ether was added to the residue and the precipitated solid was filtered and dried to obtain the target compound (514 mg, yield: 25%) as a white solid.
$^1$H-NMR (400 MHz, DMSO-$d_6$) δppm: 1.92-2.01 (1H,m), 2.16 (3H,s), 2.18 (3H,s), 3.52-3.55 (4H,m), 3.77 (2H,d,J=6 Hz), 4.52 (2H,t,J=5 Hz), 4.67 (1 H,d,J=14 Hz), 4.75 (1 H,d,J=14 Hz), 7.28 (2H,dd,J=3, 6 Hz), 7.63 (2H,brs), 8.14 (1 H,s)
(15c): Sodium Salt of 2-[[[4-[3-hydroxy-2-(hydroxymethyl)propoxy]-3,5-dimethylpyridin-2-yl]methyl]sulfinyl]-1*H*-benzimidazole
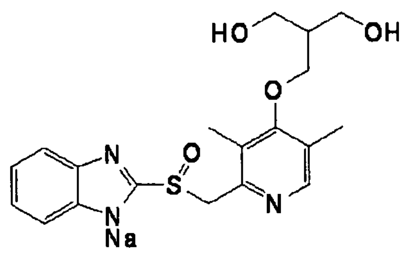

To a mixture of 2-[[[2-[(1*H*-benzimidazol-2-ylsulfinyl)methyl]-3,5-dimethylpyridine-4-yl]-oxy]methyl] propane-1,3-diol obtained in (9b) above (514 mg, 1.32 mmol) and ethanol (40 mL) was added a 1 N aqueous sodium hydroxide solution (1.32 mL, 1.32 mmol for a concentration of 1.001 M) at room temperature, which was stirred for 1 hour. The reaction mixture was concentrated and ethanol was added to the residue followed by evaporating the solvent under a reduced pressure. Moreover, ethanol was again added to the residue and the solvent was again evaporated under a reduced pressure. Diethyl ether was then added to the residue followed by sonication with ultrasonic waves and filtration and drying of the solid that precipitated in the reaction mixture. The solid was further crushed in a mortar and the solvent was further evaporated with a vacuum pump to obtain the target compound (387 mg, yield: 71 %) as a white solid.
$^1$H-NMR (400 MHz, DMSO-$d_6$) δppm: 1.94-2.00 (1H,m), 2.18 (3H,s), 2.22 (3H,s), 3.54 (4H,brs), 3.78 (2H,d,J=6 Hz), 4.39 (1 H,d,J=13 Hz), 4.53 (2H,brs), 4.76 (1 H,d,J=13 Hz), 6.89 (2H,dd,J=3, 6 Hz), 7.24 (2H,dd,J=3, 6 Hz), 8.18 (1 H,s)

(Example 16) 3-[3-Hydroxy-2-(hydroxymethyl)propoxy]-2,4-dimethyl-5*H*-pyrido [1',2',4,5][1,2,4]thiadiazino [2,3-a]benzimidazolium hexafluorophosphate

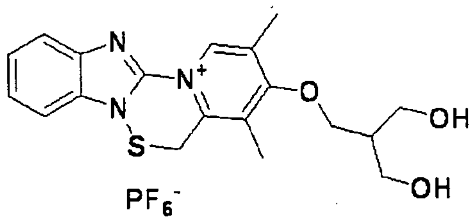

To a mixture of 2-[[[3-methyl-4-(1,5,9-trioxaspiro[5.5]undeca-3-ylmethoxy)pyridin-2-yl]methyl]sulfinyl]-1H-benzimidazole (429 mg, 1 mmol) and methanol (5 mL) was added hexafluorophosphoric acid (295 μL, 2.01 mmol for a content of 60%), which was stirred for 7 minutes at 40°C, followed by further stirring for 30 minutes while cooling with an ice bath. The solid that precipitated in the reaction mixture was filtered out and the resulting solid was washed with ether followed by evaporating the solvent under a reduced pressure to obtain the target compound (425 mg, yield: 82%) as a yellow solid.
$^1$H-NMR (400 MHz, $CD_3CN$) δppm: 2.18-2.26 (1H,m), 2.53 (3H,s), 2.64 (3H,s), 3.71-3.79 (4H,m), 4.65 (2H,d,J=6 Hz), 4.85 (2H,s), 7.84 (1H,dd,J=8 Hz), 7.57 (1H,dd,J=8, 8 Hz), 7.63 (1 H,d,J=8 Hz), 7.86 (1 H,d,J=8 Hz), 9.30 (1 H,s)

(Example 17) 3-[3-(2,2-Dimethyl-1,3-dioxan-5-yl)methoxy-2,4-dimethyl-5*H*-pyrido[1',2',4,5][1,2,4]thiadiazino [2,3-a]benzimidazolium chloride

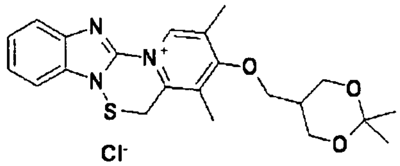

To a sodium salt of an optical isomer having a short retention time of 2-[[[4-(2,2-dimethyl-1,3-dioxan-5-yl) methoxy-3,5-dimethylpyridin-2-yl]methyl]sulfinyl]-1 H-benzimidazole (1.25 mg) was added 0.1 mol/L hydrochloric acid (2 mL). When the mass spectrum was immediately measured (within 1 minute) under the conditions indicated below, molecular ion was detected at an m/z of 412, thereby confirming formation of the target compound (see FIG 1).

Measurement Conditions:
Apparatus: SSQ7000 (ThermoQuest Corp.)
Ionization method: ESI(+)
Sample injection: Flow injection
Scanning method: Regular scan
Scanning range: m/z 50 to 1000

(Test Example 1) Gastric Acid Secretion Inhibitory Effects in Dogs Fitted with Chronic Gastric Fistula Tube

(1) Method

The gastric acid secretion inhibitory and duration of gastric acid secretion inhibitory action of compounds of the examples were assessed using large dogs fitted with a chronic gastric fistula tube (body weights: approx. 14 to 19 kg). The experiment was conducted for 2 days. On the first day, gastric juices were collected every 20 minutes under conditions of continuous intravenous administration of histamine (50 or 75 μg/kg/h) for 3 hours. A compound of the examples dissolved or suspended in 0.5% methyl cellulose solution was administered via a catheter implanted in the duodenum at the rate of 0.1 ml/kg one hour after the start of histamine administration. The gastric acid secretion inhibitory action of the compound of the examples was assessed over the next two hours. On the second day (24 hours after administration of the compound of the examples), gastric juices were collected every 20minutes under conditions of continuous intravenous administration of histamine for 2 hours followed by assessment of the duration of gastric acid secretion inhibitory action. After measuring the amount of gastric juices, 0.5 ml of gastric juice were sampled and titrated to neutrality to a pH of 7.0 with 0.04 mol/L aqueous sodium hydroxide solution to measure acid concentration. The amount of gastric acid discharged was determined by multiplying the amount of gastric juice by the acid concentration. Gastric acid secretion inhibitory action was evaluated as the rate of gastric acid secretion inhibition (%) on day 1. Gastric acid secretion inhibitory action (%) was determined according to the formula indicated below:

$$\text{Gastric acid secretion inhibitory action (\%)} = (A-B)/A \times 100$$

wherein

[A]: amount of gastric acid discharged for 20 minutes from 40 minutes to 1 hour after the start of administration of histamine
[B]: amount of gastric acid discharged for 20 minutes from 1 hour 40 minutes to 2 hours after the start of administration of the compound of the examples.
The duration of gastric acid secretion inhibitory action was evaluated as the rate of gastric acid secretion inhibition (%) on day 2. The duration of gastric acid secretion inhibitory action (%) was determined according to the formula indicated below:

$$\text{Duration of gastric acid secretion inhibitory action (\%)} = (C-D)/C \times 100$$

wherein
[C]: Total amount of gastric acid discharged from start of administration of histamine on day 1 to one hour later
[D]: Total amount of gastric acid discharged from start of administration of histamine on day 2 to one hour later.

(2) Results

Table 1 indicates the results of determining gastric acid secretion inhibitory effects in dogs fitted with chronic gastric fistula tube.
[Table 1]

| | Dosage (mg/kg, i.d.) | Gastric acid secretion inhibitory action (%) | Duration of gastric acid secretion inhibitory action (%) |
|---|---|---|---|
| Example 9 | 1.6 | 100 | 89 |
| Example 13 | 1.6 | 100 | 84 |

On the basis of the results shown in Table 1, each of the compounds of Examples 9 and 13 were determined to have satisfactory gastric acid secretion inhibitory action and duration of gastric acid secretion inhibitory action.

(Test Example 2) $H^+,K^+$-ATPase Activity Inhibitory Action

(1) Preparation of $H^+,K^+$-ATPase
$H^+,K^+$-ATPase was prepared from a fundic gland area of fresh hog gastric mucosa using a variation of the Chang's method [Biochim. Biophys. Acta 464, 313 (1977)].
(2) Measurement of $H^+$, $K^+$-ATPase activity

(1) The sodium salt of 2-[[[4-[3-hydroxy-2-(hydroxymethyl)propoxy]-3,5-dimethylpyridin-2-yl]methyl]sulfinyl]-1*H*-benzimidazole (Example 15) and (2) 3-[3-hydroxy-2-(hydroxymethyl)propoxy]-2,4-dimethyl-5*H*-pyrido[1',2',4,5][1,2,4] thiadiazino[2,3-a]benzimidazolium hexafluorophosphate (Example 16) were selected for the test compounds, and each was used after dissolving in methanol.
10 μL aliquots of various concentrations of the test compounds or solvent were mixed into 200 μL of 50 μg/mL $H^+,K^+$-ATPase, 100 μL of 10 mmol/L Pipes-Tris (pH 6.1) and 190 μL of distilled water followed by incubating for 30 minutes at 37°C, adding 150 μL of 100 mmol/L KCl (or distilled water) and 10 μL of 100 μg/mL gramicidin, and further incubating for 10 minutes. 100 μL of 400 mmol/L Tris-HCl (pH 7.4), 140 μL of distilled water and 100 μL of 30 mmol/L Mg-ATP were then added to initiate the ATPase reaction followed by incubating for 10 minutes (total reaction solution: 1 mL). The amount of inorganic phosphoric acid that was released during this 10 minute period was measured in compliance with the method of Yoda and Hokin (Biochem. Biophys. Res. Commun., 40, 880, 1970). The difference between ATPase activity in the presence of KCl and ATPase activity in the absence of KCl was taken to represent $H^+,K^+$-ATPase activity (μmol/mg protein/h).

Residual enzyme activity at each concentration of test compound was determined as the amount of $H^+,K^+$-ATPase activity as a percentage of the $H^+,K^+$-ATPase activity of a control group (solvent addition). $IC_{50}$ values were then determined from the relationship between test compound concentration and residual enzyme activity. The $IC_{50}$ (μmol/L) values of the above-mentioned test compounds (1) and (2) were 2.03 and 0.22, respectively.

Industrial Applicability

Since compounds according to the present invention have superior gastric acid secretion inhibitory action, have ample long-lasting gastric acid secretion inhibitory action, are safe and have suitable physicochemical stability, they can be used as useful pharmaceuticals, and particularly as pharmaceuticals for the treatment or prophylaxis of diseases or symptoms caused by gastric acid.

## Claims

**1.** A compound represented by the following general formula (1a):

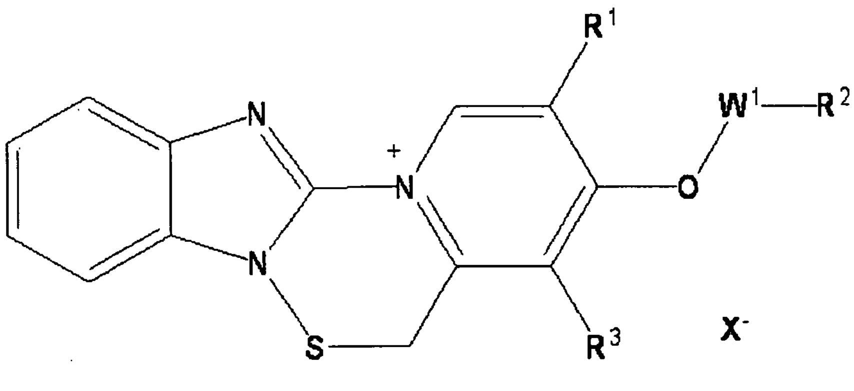

(1a)

(wherein R1 and R3 may be the same or different from and represent a hydrogen atom or a C1-C6 alkyl group, R2 represents a group represented by the following formula:

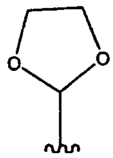 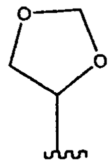 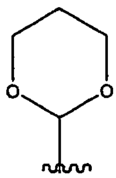 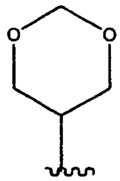 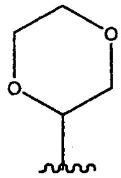 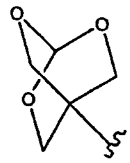

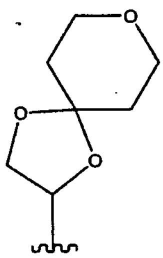 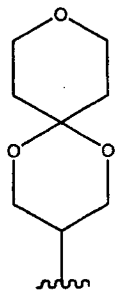 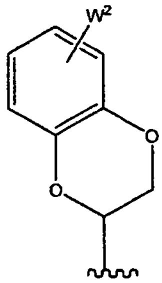 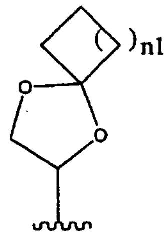 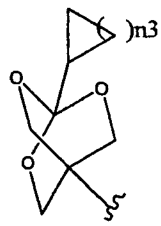

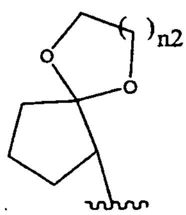 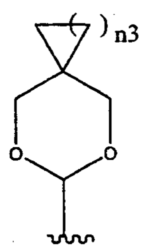 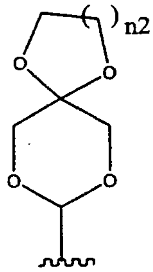 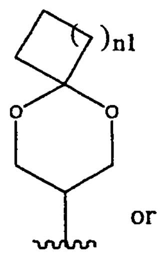 or 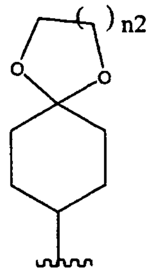 ,

in which the group may have one to four groups selected from group A1 below, or a C2-C6 alkyl group which may have one to three hydroxyl groups,

W1 represents a single bond or a linear or branched C1-C8 alkylene group,

W2 represents a hydrogen atom, a C1-C6 alkyl group or a halogen atom (provided that one to three same or different W2 groups are able to be present on a benzene ring),

n1 represents 1 to 5, n2 represents 1 to 4, n3 represents 1 to 6, and

$X^-$ represents $Cl^-$, $Br^-$, $I^-$, $BF_4^-$, $PF_6^-$, $HSO_4^-$, $SO_4^{2-}$, $CH_3SO_3^-$, 4-Me-Ph-$SO_3^-$, $PO_4^{3-}$, $ClO_4^-$ or $AuCl_4^-$);
<Group A1 >
a halogen atom, a C1-C6 alkyl group, a C1-C6 alkoxy group, a C1-C6 haloalkyl group, a C1-C6 alkoxy-C1-C6 alkyl group and a hydroxyl group.

**2.** The compound according to Claim 1, wherein R2 represents a group represented by the following formula:

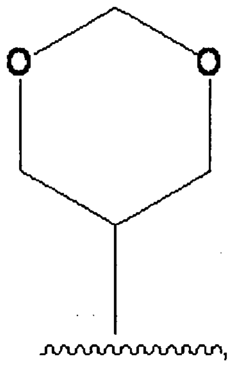

in which the group may have one or two C1-C6 alkyl groups, or a C3-C6 alkyl group having two hydroxyl groups.

**3.** The compound according to Claim 1, wherein formula W1-R2 represents formula below:

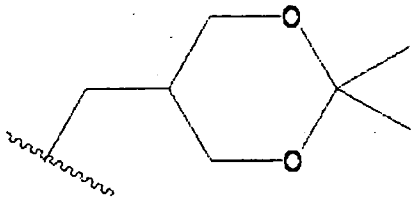

or

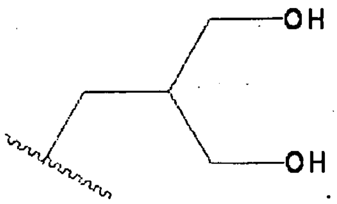

**4.** The compound according to any one of Claims 1 to 3, wherein R1 and R3 represent methyl groups.

**5.** The compound according to any one of Claims 1 to 4, wherein $X^-$ represents $Cl^-$, $Br^-$, $BF_4^-$, $PF_6^-$, $PO_4^{3-}$ or $ClO_4^-$.

**6.** A compound represented by the following formula (1 b), or a salt thereof:

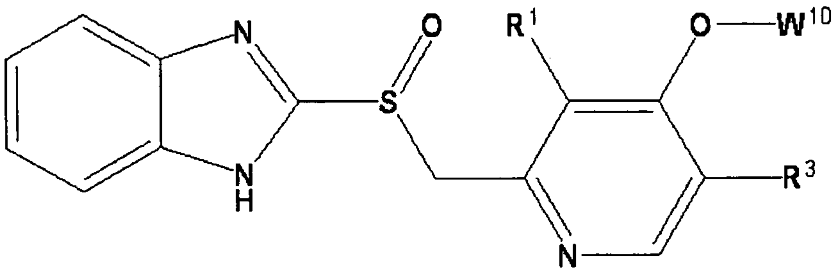

(1b)

(wherein R1 and R3 may be the same or different and represent a hydrogen atom or C1-C6 alkyl group, and W10 represents a group represented by the following formula:

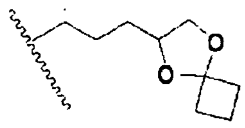 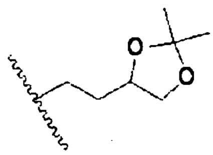 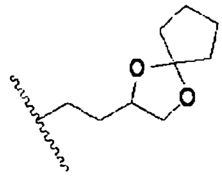

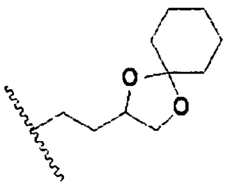 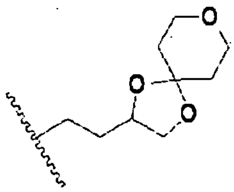 or 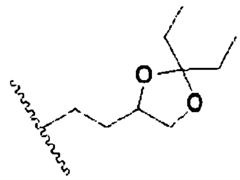

or C2-C6 alkyl group having one to three hydroxyl groups).

**7.** The compound or the salt thereof according to Claim 6, wherein the compound is a compound selected from the following group of compounds:

2-[[[4-[3-(5,8-dioxaspiro[3,4]octa-6-yl)propoxy]-3-methylpyridin-2-yl]methyl] sulfinyl]-1*H*-benzimidazole,
2-[[[4-[2-(5,8-dioxaspiro[3,4]octa-6-yl)ethoxy]-3-methylpyridin-2-yl]methyl] sulfinyl]-1*H*-benzimidazole,
2-[[[4-[2-(1,4-dioxaspiro[4,4]nona-2-yl)ethoxy]-3-methylpyridin-2-yl]methyl] sulfinyl]-1*H*-benzimidazole,
2-[[[4-[2-(1,4-dioxaspiro[4,5]deca-2-yl)ethoxy]-3-methylpyridin-2-yl]methyl] sulfinyl]-1*H*-benzimidazole,
2-[[[3-methyl-4-[2-(1,4,8-trioxaspiro[4,5]deca-2-yl)ethoxy]pyridin-2-yl]methyl]sulfi nyl]-1*H*-benzimidazole,
2-[[[4-[2-(2,2-diethyl-1,3-dioxolan-4-yl)ethoxy]-3-methylpyridin-2-yl]methyl] sulfinyl]-1*H*-benzimidazole, and
2-[[[4-[3-hydroxy-2-(hydroxymethyl)propoxy]-3,5-dimethylpyridin-2-yl]methyl] sulfinyl]-1*H*-benzimidazole.

FIG. 1

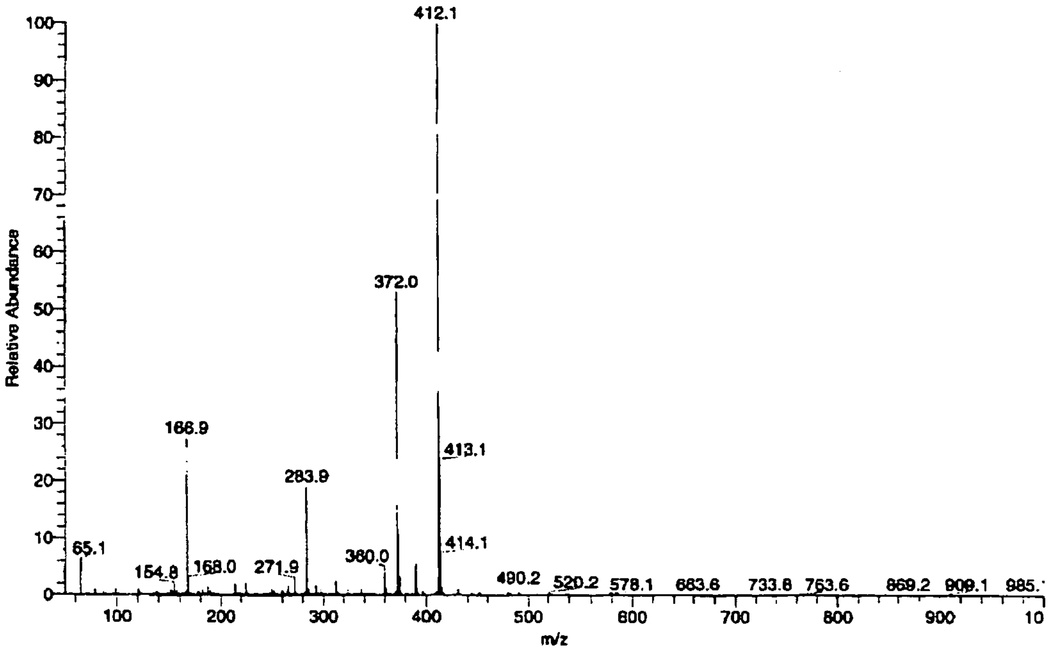
412.1
372.0
166.9
283.9
413.1
414.1
65.1
154.8
168.0
271.9
360.0
490.2
520.2
578.1
683.6
733.8
763.6
869.2
909.1
985.
Relative Abundance
m/z

**INTERNATIONAL SEARCH REPORT**

International application No.
PCT/JP2007/069863

A. CLASSIFICATION OF SUBJECT MATTER
*C07D401/12*(2006.01)i, *A61K31/4439*(2006.01)i, *A61K31/549*(2006.01)i, *A61P1/04*
(2006.01)i, *A61P31/04*(2006.01)i, *A61P43/00*(2006.01)i, *C07D405/14*(2006.01)i,

B. FIELDS SEARCHED

Minimum documentation searched (classification system followed by classification symbols)
C07D401/12, A61K31/4439, A61K31/549, A61P1/04, A61P31/04, A61P43/00, C07D405/14, C07D493/10, C07D513/14, C07D519/00

Documentation searched other than minimum documentation to the extent that such documents are included in the fields searched

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 02-049792 A (Eisai Co., Ltd.),<br>20 February, 1990 (20.02.90),<br>Full text; particularly, Claim 1; example 11; general formula (II)<br>& WO 89/10927 A1 & EP 374261 A1<br>& JP 2779206 B2 & JP 02-049781 A<br>& JP 2766309 B2 & CA 1339369 C<br>& US 5162317 A & FI 9000098 A<br>& FI 93121 B & FI 93121 C<br>& NO 9000116 A & NO 177750 B<br>& NO 177750 C & DK 9000076 A<br>& US 5239079 A & FI 98064 B<br>& FI 98064 C & NO 9501979 A | 1,4,5<br>1-5 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>28 December, 2007 (28.12.07) | Date of mailing of the international search report<br>15 January, 2008 (15.01.08) |
|---|---|
| Name and mailing address of the ISA/<br>Japanese Patent Office<br>Facsimile No. | Authorized officer<br><br>Telephone No. |

Form PCT/ISA/210 (second sheet) (April 2007)

INTERNATIONAL SEARCH REPORT

International application No.
PCT/JP2007/069863

C (Continuation). DOCUMENTS CONSIDERED TO BE RELEVANT

| Category* | Citation of document, with indication, where appropriate, of the relevant passages | Relevant to claim No. |
|---|---|---|
| Y | LINDBERG, Per et al., The mechanism of action of the gastric acid secretion inhibitor omeprazole, Journal of Medicinal Chemistry, 1986, vol.29, no.8, p.1327-9, full text, particularly, Scheme I | 1-5 |
| Y | JP 59-181277 A (Aktiebolaget Haessle), 15 October, 1984 (15.10.84), Full text; particularly, Claims 1, 6 to 8<br>& DE 3404610 A1 & SE 8400688 A<br>& BE 898880 A1 & DK 8400591 A<br>& FI 8400547 A & NO 8400504 A<br>& GB 2134523 A & GB 2134523 B<br>& AU 8424456 A & AU 578891 B2<br>& NL 8400446 A & ZA 8401011 A<br>& FR 2543551 A1 & FR 2543551 B1<br>& AT 8400435 A & AT 386825 B<br>& CH 666892 A5 & GB 2174988 A<br>& GB 2174988 B & SE 8700498 A<br>& SE 8700499 A & NO 8802001 A<br>& US 5039806 A | 1-7 |
| Y | JP 05-507713 A (Aktiebolaget Astra), 04 November, 1993 (04.11.93), Full text; particularly, Claims 1, 12 to 13; page 3, upper left column<br>& WO 91/19711 A1 & ZA 9104296 A<br>& ZA 9104297 A & CA 2083714 A1<br>& AU 9180097 A & AU 649453 B2<br>& EP 535081 A1 & HU 62881 A2<br>& PL 166209 B1 & RO 110493 B1<br>& CN 1058212 A & CZ 279772 B6<br>& NO 9204775 A & LT 3952 B<br>& LT 3977 B | 1-7 |
| Y | JP 61-178919 A (Banyu Pharmaceutical Co., Ltd.), 11 August, 1986 (11.08.86), Full text; particularly, Claim 1; examples (Family: none) | 1-5 |

Form PCT/ISA/210 (continuation of second sheet) (April 2007)

**INTERNATIONAL SEARCH REPORT**

International application No.
PCT/JP2007/069863

C (Continuation). DOCUMENTS CONSIDERED TO BE RELEVANT

| Category* | Citation of document, with indication, where appropriate, of the relevant passages | Relevant to claim No. |
|---|---|---|
| X | JP 01-006270 A (Eisai Co., Ltd.),<br>10 January, 1989 (10.01.89),<br>Full text; particularly, Claim 1, (9); table 1, compounds 10, 22<br>& EP 268956 A2 & EP 268956 A3<br>& EP 268956 B1 & EP 268956 B2<br>& FI 8704709 A & FI 90544 B<br>& FI 90544 C & NO 8704477 A<br>& NO 170932 B & NO 170932 C<br>& DK 8705758 A & DK 171024 B1<br>& DD 290192 A5 & AU 8781138 A<br>& AU 594836 B2 & HU 45524 A2<br>& HU 198475 B & CA 1265138 A1<br>& SU 1780535 A3 & CN 87107777 A<br>& CN 1018923 B & JP 06-074272 B2<br>& EP 475456 A1 & EP 475456 B1<br>& AT 103912 T & ES 2061471 T3<br>& EP 654471 A1 & EP 654471 B1<br>& EP 786461 A1 & AT 163011 T<br>& ES 2112260 T3 & AT 168111 T<br>& ES 2118457 T3 & US 5045552 A<br>& RU 2035461 C1 & JP 05-247035 A<br>& JP 2544567 B2 & DK 9301318 A<br>& JP 07-291967 A & JP 2576843 B2<br>& US 5840910 A & DK 9501442 A<br>& DK 174366 B1 & US 5998445 A | 6 |
| A | WO 2004/077367 A1 (Zeria Pharmaceutical Co., Ltd.),<br>10 September, 2004 (10.09.04),<br>Full text; particularly, Claim 1; page 2<br>& JP 2006-188432 A | 1-7 |
| E,X | WO 2007/122686 A1 (Eisai R&D Management Kabushiki Kaisha),<br>01 November, 2007 (01.11.07),<br>Full text; particularly, Claim 1<br>& WO 2007/122755 A1 | 6,7 |
| P,X | WO 2006/112442 A1 (Eisai R&D Management Kabushiki Kaisha),<br>26 October, 2006 (26.10.06),<br>Full text; particularly, Claim 1<br>& US 2007/010542 A1 & US 2007/015782 A1 | 6,7 |

Form PCT/ISA/210 (continuation of second sheet) (April 2007)

**INTERNATIONAL SEARCH REPORT**

International application No.
PCT/JP2007/069863

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet.

1. ☒ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (April 2007)

**INTERNATIONAL SEARCH REPORT**

International application No.
PCT/JP2007/069863

Continuation of Box No.III of continuation of first sheet(2)

The inventions of claims 1-5 (i) relate to a compound represented by the general formula (1a); and the inventions of claims 6 and 7 (ii) relate to a compound represented by the general formula (1b).

A common technical feature between the inventions (i) and the inventions (ii) appears to reside in a chemical structure which has a thiomethyl partial structure (wherein the term "partial structure" means to contain a structural moiety constituting the ring, too) on position-2 in a pyridine ring moiety, has an oxyalkyloxy partial moiety (wherein the term "partial structure" is as stated above) on position-4 in the pyridine ring moiety and has "a hydrogen" or "a C1-C6 alkyl group" on both position-3 and position-5 in the pyridine ring moiety. However, the common technical feature is already known (if needed, see JP 01-006270 A (Eisai Co., Ltd.) 10 January, 1989 (10.01.89), Compound Nos.10 and 22 in Table 1 and so on). Therefore, it cannot be considered that these inventions (i) and (ii) are so related as to form a single general inventive concept.

Consequently, the present international application includes the above-stated two groups of inventions (i) and (ii).

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9119712 A
- JP S59181277 B
- JP S617281 B
- WO 9602535 A
- WO 200183473 A
- WO 2004087702 A
- WO 2004052881 A
- WO 2004052882 A


### Non-patent literature cited in the description


- *Digestion,* 1992, vol. 51 (1), 59-67
- *Journal of Medicinal Chemistry,* 1986, vol. 29, 1327-1329
- *Adv. Synth. Catal.,* 2005, vol. 347, 19-31
- *Chem. Rev.,* 2003, vol. 103, 3651-3705
- *Tetrahedron Lett.,* 2004, vol. 45, 9249-9252
- *Angew. Chem. Int. Ed.,* 2004, vol. 43, 4225-4228
- *Tetrahedron Asymmetry,* 2003, vol. 14, 407-410
- *J. Med. Chem.,* 1998, vol. 41, 1777-1788
- *Tetrahedron,* 2003, vol. 59, 5861-5868
- *Biochim. Biophys. Acta,* 1977, vol. 464, 313
- **Yoda ; Hokin.** *Biochem. Biophys. Res. Commun.,* 1970, vol. 40, 880